# EUROPEAN PATENT SPECIFICATION

(11) **EP 2 833 740 B1**
(45) Date of publication and mention of the grant of the patent: **14.09.2016**
(21) Application number: 13731876.2
(22) Date of filing: 15.03.2013
(51) Int. Cl.: A23L 33/10, A23L 33/155

(54) **COMPOSITIONS COMPRISING OMEGA-3 FATTY ACIDS AND VITAMIN D FOR ACNE VULGARIS AND/OR ECZEMA, AND METHODS AND USES THEREOF**
ZUSAMMENSETZUNGEN MIT OMEGA-3-FETTSÄUREN UND VITAMIN D FÜR AKNE VULGARIS UND/ODER EKZEME SOWIE VERFAHREN UND VERWENDUNGEN DAVON
COMPOSITIONS COMPRENANT DES ACIDES GRAS OMÉGA-3 ET DE LA VITAMINE D DESTINÉES À LUTTER CONTRE L'ACNÉ VULGAIRE ET/OU L'ECZÉMA, ET MÉTHODES ET UTILISATIONS ASSOCIÉES

(30) Priority: 04.04.2012 US 201261620405 P
(43) Date of publication of application: 11.02.2015
(73) Proprietor: Pronova BioPharma Norge AS, 1327 Lysaker (NO)
(72) Inventor: FRASER, David, A., 1365 Blommenholm (NO); GARRAS, Alexis, 0198 Oslo (NO)
(74) Representative: Zacco Norway AS
(86) International application number: PCT/IB2013/001235
(87) International publication number: WO 2013/150386

(56) References cited:
- WO-A2-03/017945
- JP-A- 2008 301 780
- US-A- 5 312 834
- US-A1- 2007 141 138

## Description

The present disclosure relates generally to food supplement, dietary supplement, nutritional supplement, over-the-counter (OTC) supplement, medical food, or pharmaceutical grade supplement compositions comprising omega-3 fatty acids and vitamin D for use in improving at least one parameter associated with acne vulgaris and/or eczema.

### Acne vulgaris

Acne vulgaris is a multifactorial disease characterized by abnormal ductal keratinization resulting in comedogenesis, increased sebum production resulting in seborrhea, abnormalities of the microbial flora, and inflammation. Given the multifactorial pathogensis and lack of understanding of a singular precipitating factor, optimal therapy should be designed to target multiple pathways.

The combination of oral vitamin D and at least one fatty acid chosen from eicosapentaenoic acid (EPA) and docosahexaenoic acid (DHA), as disclosed herein, for the improvement of at least one parameter associated with acne vulgaris achieves this aim, by simultaneously targeting the microbial, inflammatory, and fibrotic aspects of the disease. This will modify both the active phase of the disease whilst minimizing the chronic aesthetic disfigurement associated with scarring. Each component (omega-3 lipids and vitamin D) exerts unique yet overlapping and complementary effects.

### Eczema

Eczema is a form of dermatitis, or inflammation of the epidermis, which may also be called dermatitis eczema and eczematous dermatitis. The term eczema is broadly applied to a range of persistent skin conditions. An eczema diagnosis often implies atopic dermatitis (which is common in children and teenagers) but, without proper context, may refer to any kind of dermatitis. There are several different types of dermatitis, which generally share an allergic reaction to specific allergens.

In some instances, dermatitis and eczema are synonyms, while in other instances dermatitis implies an acute condition and eczema a chronic one. The two conditions are often classified together. The terms "eczema" and "dermatitis" are used interchangeably in the present application. These include dryness and recurring skin rashes which are characterized by one or more of these symptoms: redness, skin edema (swelling), itching and dryness, crusting, flaking, blistering, cracking, oozing, or bleeding. Itchy rash is particularly noticeable on head and scalp, neck, inside of elbows, behind knees, and buttocks. There are several types of eczematous disorders, for example atopic eczema, asteatotic eczema, contact eczema, allergic contact eczema, seborrheic eczema, nummular eczema, dyshidrotic eczema, dermatitis herpetiformis, stasis dermatitis and neurodermatitis.

### BACKGROUND

WO 03/017945 A2 is directed to compositions of unsaturated fatty acids for use by women and their children during stages of preconception, pregnancy and lactation to improve the health of the woman and the child. Different ratios of omega-6 highly unsaturated fatty acids (HUFAs) and omega-3 HUFAs are provided for the different stages. The omega-3 HUFA comprises at least one of DHA and docosapentaenoic acid (DPA) (n-3). The product provided is a food and the levels of HUFA source in the food ranges from about 0.1 to 20 %, and preferably the total fatty acids comprise from about 1% to about 15 % DHA. The examples provide compositions comprising omega-6 fatty acids (arachidonic acid (ARA)), DHA and vitamin D in the amount of 200 IU, in addition to other vitamins and minerals. There is no teaching that the composition comprises a fatty acid oil mixture of 25-100 percent of EPA and/or DHA. Further, there is no teaching or suggestion of using the composition in treatment of skin problems, such as acne/eczema.

JP 2008 301780 A discloses a food for atopic dermatitis comprising an acidic xylo-oligosaccharide and a vitamin and/or a polyunsaturated fatty acid. There is no indication that the food disclosed comprises a fatty acid oil mixture of 25-100 percent of EPA and/or DHA.

US 2007/141138 is directed to pharmaceutical formulations comprising EPA and DHA, possibly in combination with vitamins or herbal supplements. The formulation may be used in treatment of a long list of different diseases and disorders, also atopic dermatitis. However, there is no teaching or suggestion in US 2007/141138 of combining the fatty acid oil mixture with vitamin D in treatment of atopic dermatitis.

### SUMMARY

The present disclosure concerns food supplement, dietary supplement, nutritional supplement, OTC supplement, medical food, or pharmaceutical grade supplement compositions for improving at least one parameter associated with acne vulgaris.

The present disclosure also relates to food supplement, dietary supplement, nutritional supplement, OTC supplement, medical food, or pharmaceutical grade supplement compositions for improving at least one parameter associated with eczema.

The present disclosure further relates to a method for improving at least one parameter associated with acne vulgaris and/or eczema in a subject in need thereof.

The present disclosure also relates to a method for improving at least one parameter associated with acne vulgaris by administration of a food supplement, dietary supplement, nutritional supplement, OTC supplement, medical food, or pharmaceutical grade supplement composition comprising omega-3 fatty acids and vitamin D.

### DESCRIPTION

The present disclosure is directed to food supplement, dietary supplement, nutritional supplement, OTC supplement, medical food, or pharmaceutical grade supplement compositions for improving at least one parameter associated with acne vulgaris and/or eczema comprising: a fatty acid oil mixture comprising from about 25% to about 100% of at least one fatty acid chosen from eicosapentaenoic acid (EPA) and docosahexaenoic acid (DHA), by weight of the fatty acid oil mixture, wherein the EPA and DHA are in a form chosen from ethyl ester, triglyceride, and free fatty acid; and vitamin D.

The present disclosure is also directed to a food supplement, dietary supplement, nutritional supplement, over-the-counter (OTC) supplement, medical food, pharmaceutical grade supplement composition for at least one aspect of skin health chosen from : improving skin health in subjects with acne, maintaining skin health in subjects with acne who are symptom-free, improving skin health in subjects with eczema, and maintaining skin health in subjects with eczema who are symptom-free comprising: a fatty acid oil mixture comprising from about 25% to about 100% of at least one fatty acid chosen from eicosapentaenoic acid (EPA) and docosahexaenoic acid (DHA), by weight of the fatty acid oil mixture, wherein the EPA and DHA are in a form chosen from ethyl ester, triglyceride, and free fatty acid; and vitamin D, wherein the DHA is present in an amount greater than 50% by weight, relative to the weight of the total fatty acids in the fatty acid oil mixture.

The present disclosure is further directed to a method for improving at least one parameter associated with acne vulgaris and/or eczema in a subject in need thereof comprising administering to the subject a food supplement, dietary supplement, nutritional supplement, OTC supplement, medical food, or pharmaceutical grade supplement composition comprising: a fatty acid oil mixture comprising from about 25% to about 100% of at least one fatty acid chosen from eicosapentaenoic acid (EPA) and docosahexaenoic acid (DHA), by weight of the fatty acid oil mixture, wherein the EPA and DHA are in a form chosen from ethyl ester, triglyceride and free fatty acid; and vitamin D.

Particular aspects of the disclosure are described in greater detail below. The terms and definitions as used in the present application and as clarified herein are intended to represent the meaning within the present disclosure. The patent and scientific literature referred to herein and referenced above is hereby incorporated by reference. The terms and definitions provided herein control, if in conflict with terms and/or definitions incorporated by reference.

The singular forms "a," "an," and "the" include plural reference unless the context dictates otherwise.

The terms "approximately" and "about" mean to be nearly the same as a referenced number or value. As used herein, the terms "approximately" and "about" should be generally understood to encompass ± 10% of a specified amount, frequency, or value.

### Fatty Acid Oil Mixture and Omega-3 Fatty Acids

Compositions of the present disclosure comprise at least one fatty acid oil mixture. The fatty acid oil mixture comprises at least one fatty acid chosen from eicosapentaenoic acid (EPA) and docosahexaenoic acid (DHA). As used herein, the term "fatty acid oil mixture" includes fatty acids, such as unsaturated (e.g., monounsaturated, polyunsaturated) or saturated fatty acids, as well as pharmaceutically-acceptable esters, free acids, mono-, di- and triglycerides, derivatives, conjugates, precursors, salts, and mixtures thereof. In some embodiments, the fatty acid oil mixture comprises fatty acids, such as omega-3 fatty acids, in a form chosen from ethyl ester and triglyceride. In other embodiments, the fatty acids of the fatty acid oil mixture are in free acid form.

As used herein, the term "omega-3 fatty acids" includes natural and synthetic omega-3 fatty acids, as well as pharmaceutically acceptable esters, free acids, triglycerides, derivatives, conjugates (see, e.g., Zaloga et al., U.S. Patent Application Publication No. 2004/0254357, and Horrobin et al., U.S. Patent No. 6,245,811, each hereby incorporated by reference), precursors, salts, and mixtures thereof.

The fatty acid oil mixture according to the present disclosure may be derived from animal oils and/or non-animal oils. In some embodiments of the present disclosure, the fatty acid oil mixture is derived from at least one oil chosen from marine oil, algae oil, plant-based oil, and microbial oil. Marine oils include, for example, fish oil, krill oil, and lipid composition derived from fish. Plant-based oils include, for example, flaxseed oil, canola oil, mustard seed oil, and soybean oil. Microbial oils include, for example, products by Martek. In at least one embodiment of the present disclosure, the fatty acid oil mixture is derived from a marine oil, such as a fish oil. In at least one embodiment, the marine oil is a purified fish oil, such as a purified tuna fish oil.

In some embodiments of the present disclosure, the fatty acids, such as omega-3 fatty acids, of the fatty acid oil mixture are esterified, such as alkyl esters and further for example, ethyl esters. In other embodiments, the fatty acids are chosen from mono-, di-, and triglycerides.

In some embodiments, the fatty acid oil mixture is obtained by a transesterification of the body oil of a fat fish species coming from, for example, anchovy or tuna oil, and subsequent physico-chemical purification processes, including urea fractionation followed by molecular distillation. In some embodiments, the crude oil mixture may also be subjected to a stripping process for decreasing the amount of environmental pollutants and/or cholesterol before the transesterification.

In another embodiment, the fatty acid oil mixture is obtained by using supercritical CO₂ extraction or chromatography techniques, for example to up-concentrate primary EPA and DHA from fish oil concentrates.

The fatty acid oil mixture of the present disclosure comprises omega-3 fatty acids, such as EPA and DHA. Further for example, in some embodiments, the fatty acid oil mixture comprises EPA and DHA in a form chosen from ethyl ester and triglyceride. In other embodiments, the fatty acid oil mixture comprises EPA and DHA in free acid form.

The fatty acid oil mixture of the present disclosure may further comprise at least one fatty acid other than EPA and DHA. Examples of such fatty acids include, but are not limited to, omega-3 fatty acids other than EPA and DHA and omega-6 fatty acids. For example, in some embodiments of the present disclosure, the fatty acid oil mixture comprises at least one fatty acid other than EPA and DHA chosen from α-linolenic acid, heneicosapentaenoic acid, docosapentaenoic acid, eicosatetraenoic acid, and octadecatetraenoic acid. Examples of further omega-3 fatty acids and mixtures thereof encompassed by the present disclosure include the omega-3 fatty acids as defined in the European Pharmacopoeia Omega-3 Triglycerides, the European Pharmacopoeia Omega-3 acid Ethyl Esters 60, or the Fish oil rich in omega-3 acids monograph. In some embodiments, the at least one fatty acid other than EPA and DHA is in a form chosen from ethyl ester and triglyceride. In other embodiments, the at least one fatty acid other than EPA and DHA is in free acid form.

Commercial examples of omega-3 fatty acids suitable for the present disclosure comprising different fatty acid mixtures (e.g., that can be in the form of triglycerides (TG), ethyl esters (EE), free fatty acid form (FA) and/or as phospholipids) include, but are not limited to: Incromega™ omega-3 marine oil concentrates such as Incromega™ E1070, Incromega™ TG7010 SR, Incromega™ E7010 SR, Incromega™ TG6015, Incromega™ EPA500TG SR, Incromega™ E400200 SR, Incromega™ E4010, Incromega™ DHA700TG SR, Incromega™ DHA700E SR, Incromega™ DHA500TG SR, Incromega™ TG3322 SR, Incromega™ E3322 SR, Incromega™ TG3322, Incromega™ E3322, Incromega™ Trio TG/EE (Croda International PLC, Yorkshire, England); EPAX6000FA, EPAX5000TG, EPAX4510TG, EPAX2050TG, EPAX7010EE, EPAX5500EE, EPAX5500TG, EPAX5000EE, EPAX5000TG, EPAX6000EE; EPAX6000TG, EPAX6000FA, EPAX6500EE, EPAX6500TG, EPAX4510TG, EPAX1050TG, EPAX2050TG, EPAX 7010TG, EPAX7010EE, EPAX6015TG/EE, EPAX4020TG, and EPAX4020EE (EPAX is a wholly-owned subsidiary of Norwegian company Austevoll Seafood ASA); MEG-3® EPA/DHA fish oil concentrates (Ocean Nutrition Canada); DHA FNO "Functional Nutritional Oil" and DHA CL "Clear Liquid" (Lonza); Superba™ Krill Oil (Aker); omega-3 products comprising DHA produced by Martek; Neptune krill oil (Neptune); cod-liver oil products and anti-reflux fish oil concentrate (TG) produced by Møllers; Lysi Omega-3 Fish oil; Seven Seas Triomega® Cod Liver Oil Blend (Seven Seas); Fri Flyt Omega-3 (Vesterålens); Pronova PURE products such as 200:500, 300:500, 240:600, 10:70, 10:70 EE/TG, 70:10 EE/TG, 50:30, 500:200 EE/TG, 400:200 EE/TG, 360:240 EE/TG, and 150:600 EE/TG, produced by Pronova; Epadel™ by Mochida; and Omthera products such as 44-55 mg/g EPA-FFA and 15-25 mg/g DHA-FFA, Amarin; >97% EPA-EE no DHA-EE, or >97w% EPA-EE and 0.5-4 mg/g; and MAXOMEGA and OMIVITAL produced by BASF.

In some embodiments of the compositions of the present disclosure, the weight ratio of EPA:DHA of the fatty acid oil mixture ranges from about 1:10 to about 10:1, from about 1:8 to about 8:1, from about 1:7 to about 7:1, from about 1:6 to about 6:1, from about 1:5 to about 5:1, from about 1:4 to about 4:1, from about 1:3 to about 3:1, or from about 1:2 to about 2:1.

In at least one embodiment of the compositions disclosed herein, the concentration by weight of DHA is higher than the concentration by weight of EPA in the fatty acid oil mixture.

In another embodiment, the fatty acid oil mixture comprises pure DHA, such as >95% DHA, in free fatty acid or ethyl ester form. In at least one such embodiment, the fatty acid oil mixture does not comprise EPA.

In yet another embodiment, the fatty acid oil mixture comprises pure EPA, such as >95% EPA, in free fatty acid or ethyl ester form.

In still another embodiment, DHA is the predominant fatty acid in the composition disclosed herein. For example, DHA is present in an amount greater than 50% by weight, relative to the weight of the total fatty acids in the fatty acid mixture.

In at least one embodiment, the fatty acid oil mixture comprises a weight ratio of EPA:DHA of 1.2:1 (about 430-about 495 mg/g EPA:about 347-about 403 mg/g DHA) and a total amount of omega-3 fatty acid ethyl esters of at least about 900 mg/g omega-3 fatty acids.

The compositions presently disclosed may further comprise at least one antioxidant. Examples of antioxidants suitable for the present disclosure include, but are not limited to, α-tocopherol (vitamin E), calcium disodium EDTA, alpha tocoferyl acetates, butylhydroxytoluenes (BHT), and butylhydroxyanisoles (BHA). Other examples of antioxidants include ascorbic acid and pharmaceutically acceptable salts thereof such as sodium ascorbate, pharmaceutically acceptable esters of ascorbic acid including fatty acid ester conjugates, propyl gallate, citric acid and pharmaceutically acceptable salts thereof, malic acid and pharmaceutically acceptable salts thereof, and sulfite salts such as sodium sulfite and mixtures thereof.

The compositions presently disclosed may further comprise at least one other vitamin other than vitamin D. Examples of other vitamins suitable for the present disclosure include, but are not limited to, Zinc and Magnesium.

### Anti-inflammatory/immunomodulatory effects of EPA/DHA

EPA and DHA supplementation may have beneficial effects upon acne vulgaris for several reasons related to their anti-inflammatory and immunomodulatory effects.

Omega-3 lipids such as EPA and DHA are ligands for peroxisome proliferator-activated receptor-alpha (PPAR-α). PPAR-α may be important in the regulation of human sebum production and the development of acne, as studies in sebocytes and human sebaceous glands indicate that PPAR agonists alter sebaceous lipid production *(see* Choi JY et al., Propionibacterium acnes stimulates pro-matrix metalloproteinase-2 expression through tumor necrosis factor-alpha in human dermal fibroblasts, J Invest Dermatol. 2008 Apr;128(4):846-54). PPAR-α regulation can also modulate the inflammation associated with acne lesions by inhibiting the expression of pro-inflammatory genes.

Further, lipoxygenase products, such as the arachadonic acid (AA) derivative, leukotriene B₄ (LTB₄), are involved in the development of inflammatory acne lesions. As an alternative substrate for 5-lipoxygenase (5-LOX), EPA generates the less potent LTB₅, and can inhibit the production of LTB4 via competitive inhibition for both PLA₂ and 5-LOX and by decreasing the expression of LTB4 receptors.

Additionally, *P. acnes* induces matrix metalloproteinase (MMP) in human dermal fibroblasts (hDF) through the activated NF-kappaB pathway *(see* Choi JY et al., Propionibacterium acnes stimulates pro-matrix metalloproteinase-2 expression through tumor necrosis factor-alpha in human dermal fibroblasts, J Invest Dermatol. 2008 Apr;128(4):846-54). Both EPA and DHA are known to potently inhibit lipopolysaccharide induced activation of NF-kappaB *(see* Mullen A et al., Anti-inflammatory effects of EPA and DHA are dependent upon time and dose-response elements associated with LPS stimulation in THP-1-derived macrophages, J Nutr Biochem. 2010 May;21(5):444-50) and may thereby inhibit pathogenic matrix remodeling in acne skin. As DHA is a more potent inhibitor of NF-kappaB activation, the present disclosure provides for a supplement with a high (<2) ratio of DHA:EPA.

### Anti-fibrotic effects of EPA/DHA

An unfortunate long-term aesthetic consequence of acne when the active phase disease has resolved itself is scarring. Although little is known regarding the effects of EPA and DHA upon dermal fibroblasts, it has been shown that treatment with EPA may attenuate atrial fibrosis, possibly via reductions in ERK phosphorylation and TGF-β1 expression *(see* Kitamura K et al., Eicosapentaenoic acid prevents atrial fibrillation associated with heart failure in a rabbit model, Am J Physiol Heart Circ Physiol. 2011 May;300(5):H1814-21). In addition to the prophylactic effects upon scar formation related to reductions in severity of the disease in its active phase, the present disclosure further provides that EPA and DHA will alter wound healing processes and attenuate the formation of hypertrophic scars or keloids.

### Food Supplement, Dietary Supplement, Nutritional Supplement, OTC Supplement, Medical Food, or Pharmaceutical Grade Supplement Composition

The present disclosure provides a food supplement, dietary supplement, a nutritional supplement, OTC supplement, medical food, or a pharmaceutical grade supplement composition comprising a fatty acid oil mixture, wherein the fatty acid oil mixture comprises from about 25% to about 100% of at least one fatty acid chosen from EPA and DHA by weight of the fatty acid oil mixture. In some embodiments, for example, the fatty acid oil mixture comprises less than 70% EPA and DHA by weight of the fatty acid oil mixture, such as less than 65%, less than 60%, less than 55%, less than 50%, less than 45%, less than 40%, or even less than 35% by weight of the fatty acid oil mixture.

In some embodiments, the fatty acid oil mixture comprises from about 25% to about 95% of the at least one fatty acid chosen from EPA and EHA, from about 35% to about 90% of the at least one fatty acid chosen from EPA and DHA, by weight of the fatty acid oil mixture, from about 40% to about 85% of the at least one fatty acid chosen from EPA and DHA, by weight of the fatty acid oil mixture, from about 40% to about 80% of the at least one fatty acid chosen from EPA and DHA, by weight of the fatty acid oil mixture, from about 40% to about 75% of the at least one fatty acid chosen from EPA and DHA, by weight of the fatty acid oil mixture, from about 40% to about 70% of the at least one fatty acid chosen from EPA and DHA, by weight of the fatty acid oil mixture, or from about 50% to about 75% of the at least one fatty acid chosen from EPA and DHA, by weight of the fatty acid oil mixture:

In at least one embodiment, the fatty acid oil mixture comprises about 60% of the at least one fatty acid chosen from EPA and DHA, by weight of the fatty acid oil mixture.

In at least one other embodiment, the fatty acid oil mixture comprises about 70% of the at least one fatty acid chosen from EPA and DHA, by weight of the fatty acid oil mixture.

In at least one other embodiment, the fatty acid oil mixture comprises about 80% of the at least one fatty acid chosen from EPA and DHA, by weight of the fatty acid oil mixture.

In at least one embodiment, the fatty acid oil mixture comprises from about 25% to about 100% of the at least one fatty acid chosen from EPA and DHA of the fatty acid oil mixture, of which at least 95% is EPA.

In at least one embodiment, the fatty acid oil mixture comprises from about 25% to about 100% of the at least one fatty acid chosen from EPA and DHA of the fatty acid oil mixture, of which at least 95% is DHA. In at least one such embodiment, the fatty acid oil mixture does not comprise EPA.

In at least one embodiment, the EPA:DHA weight ratio of the fatty acid oil mixture ranges from about 1:10 to about 1:5. In at least one other embodiment, the EPA:DHA weight ratio of the fatty acid oil mixture is about 1:7. In yet another embodiment, the EPA:DHA weight ratio of the fatty acid oil mixture ranges from about 2:5 to about 3:5.

In at least one embodiment, the fatty acid oil mixture comprises from about 80 to about 130 mg/g EPA ethyl ester or triglyceride and from about 650 to about 750 mg/g DHA ethyl ester or triglyceride. In a further embodiment, the fatty acid oil mixture comprises from about 750 mg/g to about 900 mg/g omega-3 fatty acids.

In at least one embodiment, the fatty acid oil mixture comprises at least about 60% of the at least one fatty acid in triglyceride form, by weight of the fatty acid oil mixture, and the fatty acid oil mixture comprises a greater amount of DHA than EPA.

In at least one embodiment, the food supplement, dietary supplement, nutritional supplement, OTC supplement, medical food, or pharmaceutical grade supplement composition comprises a first composition comprising the fatty acid oil mixture and a second composition comprising the vitamin D.

In another embodiment, the food supplement, dietary supplement, nutritional supplement, OTC supplement, medical food, or pharmaceutical grade supplement composition comprises a single composition comprising the fatty acid oil mixture and the vitamin D.

### Vitamin D

Vitamin D, or calciferol, is the general name for a collection of steroid-like substances including vitamin D₂ (ergocalciferol) and vitamin D₃ (cholecalciferol). The latter is the naturally occurring form used for low dose supplementation.

The term "vitamin D" used herein, means vitamin D₃. Vitamin D₃ is a secosteroid. The IUPAC name is (3β,5*Z*,7*E*)-9,10-secocholesta-5,7,10(19)-trien-3-ol. Another name is activated 7-dehydrocholesterol. The chemical structure of vitamin D₃ is as follows:

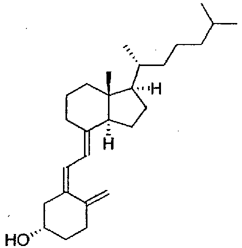

Vitamin D₃ is metabolised by the liver to 25(OH)D₃ (also known as 25 hydroxycholecalciferol, calcifediol, or calcidiol), which is then converted by the kidneys to 1,25(OH)₂D₃ (also known as 1,25 dihydroxycholecalciferol, calcitriol, or active vitamin D hormone). 25(OH)D₃, the major circulating form, has some metabolic activity, but 1,25(OH)₂D₃ is the most metabolically active.

The chemical structure of 25(OH)D₃ is as follows:

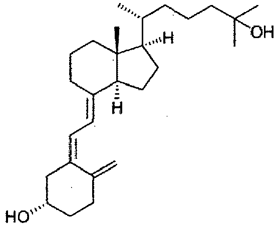

The chemical structure of 1,25(OH)₂D₃ is as follows:

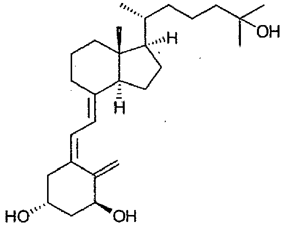

The vitamin D may, for example, be suspended or dissolved in the fatty acid oil mixture, encapsulated or micro-encapsulated in the fatty acid oil mixture, sprayed on a capsule encapsulating the fatty acid oil mixture, or cap-in-cap capsules.

The daily dose of Vitamin D₃ may range from 400-6000 International Units (IU) optionally divided into 2-4 capsules or tablets or any other formulation of the omega-3 fatty acid composition. For example, in at least one embodiment, the daily dose of Vitamin D₃ ranges from about 1000 to about 4000 IU or from about 2000 to about 4000 IU.

### Anti-microbial effects of vitamin D₃

Vitamin D₃ is known to have potent immunomodulatory and anti-microbobial effects *(see* White JH. Vitamin D metabolism and signaling in the immune system, Rev Endocr Metab Disord. 2012 Mar;13(1):21-9). Supplementation with vitamin D₃ stimulates the generation of anti-microbial proteins (AMPs), such as cathelicidin, which could potentially reduce the proliferation of *Propionibacterium acnes (P. acnes).* A central pathogenic factor of acne is the proliferation of normal flora, such as, for example, *P. acnes.* Although acne is not an infectious disease, the importance of *P. acnes* is suggested by the successful antibiotic therapy of acne and by the observation that the presence of resistant strains of *P. acnes* may be associated with therapeutic failure.

The fact that vitamin D receptors (VDR) are expressed on 2 cell types that play a role in the pathogenesis of acne vulgaris-keratinocytes and sebocytes-strongly supports the possibility that oral vitamin D₃ may exert microbicidal effects that limit the proliferation of *P. acnes.* The anti-inflammatory and immunomodulatory effects of vitamin D₃ offer additional therapeutic effects by limiting immune responses to other innocuous agents such as skin lipids and their metabolic derivatives.

### Synergistic Effect

Optimal treatment of acne vulgaris necessitates the targeting of multiple inter-related steps in the disease process. The anti-microbial effects of vitamin D combined with the anti-inflammatory/immunodulatory and anti-fibrotic effects of EPA and DHA achieves this by limiting the proliferation of *P. acnes,* by reducing inflammatory responses to *P. acnes* and other innocuous agents, and by reducing scar formation associated with chronic inflammation.

As many inflammatory skin diseases are associated with both an infectious and inflammatory component, e.g., seborrheic dermatitis and acne vulgaris, the ideal treatment should target both anti-microbial and anti-inflammatory aspects of the disease. As it is often unclear in a number of skin disorders whether pathogenic microbial changes in the skin/sebaceous gland initiate an inflammatory response or whether an overactive inflammatory response facilitates microbial changes, it also makes sense to target both the inflammatory and microbial components to break this self-perpetuating cycle. For example, in acne vulgaris, the anti-microbial effects of vitamin D could limit the proliferation of P. acnes whilst the anti-inflammatory/immunodulatory effects of EPA/DHA could reduce the inflammatory responses to P. acnes and other innocuous agents. The anti-fibrotic effects of EPA/DHA could also reduce scar formation associated with chronic inflammation. The same principle applies to other skin disorders where infectious/inflammatory processes interact to create a chronic self-perpetuation process.

### Usual (Common) Treatment of Acne

The composition disclosed herein may be administered to reduce a dosage of usual (common) treatment of acne. Usual (common) acne treatments include, for example, over-the-counter topical treatments, prescription topical treatments, antibiotics, antimicrobials, retinoids, isotretinoin, oral contraceptives, laser and light therapies, and cosmetic procedures.

### Over-the-counter topical treatments

Over-the-counter topical treatments include, for example, lotions. Acne lotions may dry up the oil, kill bacteria, and promote sloughing of dead skin cells. Over-the-counter (OTC) lotions are generally mild and may contain benzoyl peroxide, sulfur, resorcinol, salicylic acid, or sulfur as their active ingredient. These products can be helpful for very mild acne. OTC acne medications may cause initial side effects - such as skin irritation, dryness and flaking - that often improve after the first month of therapy.

### Prescription topical treatments

A variety of prescription medications are used today to clear acne. Topical (applied to the skin) medications may be prescribed for mild to severe acne. Systemic (works internally) therapy is needed to treat severe acne and may be used for moderate cases. These medications, which play an important role in acne treatment, attack the different factors that lead to acne. To achieve long-term control and resolution, dermatologists may combine therapies. The following describes the prescription medications used in the United States to treat acne.

Topical treatments available by prescription may be obtained via a doctor or dermatologist. Tretinoin (Avita, Retin-A, others), adapalene (Differin) and tazarotene (Tazorac, Avage) are examples of topical prescription products derived from vitamin A. They work by promoting cell turnover and preventing plugging of the hair follicles. A number of topical antibiotics also are available. They work by killing excess skin bacteria.

Often, a combination of such products is required to achieve optimal results. A number of benzoyl peroxide and antibiotic combination medications are available, including different dose combinations of benzoyl peroxide and clindamycin (Benzaclin, Duac, Acanya) and benzoyl peroxide and erythromycin (Benzamycin). Dapsone gel (Aczone) is a newer acne treatment that's particularly effective in treating inflammatory acne. Prescription topical treatments for acne may cause skin side effects, such as stinging, burning, redness or peeling.

### Oral Antibiotics

For moderate to severe acne, a short course of prescription oral antibiotics to reduce bacteria and fight inflammation has been a mainstay of therapy for years. Like topical antimicrobials, oral antibiotics work to reduce the *P. acnes* population (a contributing factor in acne), which, in turn, decreases inflammation. Treatment with oral antibiotics usually begins with a higher dosage, which is reduced as acne resolves. Generally, antibiotics are prescribed for six months or less.

Since oral antibiotics were first used to treat acne, antibiotic resistance has increased significantly in people with acne because over time, the *P*. *acnes* bacteria can become resistant to the antibiotic being used to treat it. When resistance occurs, acne is no longer controlled. Another antibiotic or alternative treatment can be prescribed. Numerous studies support the effectiveness of the following oral broad-spectrum antibiotics, which are used to treat acne in the United States: erythromycin, tetracycline and derivatives.

Erythromycin is effective against a broad spectrum of bacteria, including *P. acnes.* The most common side effect is irritation of the gastrointestinal tract.

Tetracycline and derivatives reduce the papules and pustules (inflammatory lesions) of acne. These medications should not be taken by children younger than 8 years of age because they can affect growth and stain teeth. They should also not be taken by a woman who is pregnant or breast feeding. During pregnancy and breastfeeding, tetracyclines can affect the development of the child's bones and teeth, leading to skeletal defects. A typical tetracycline regimen for treating moderate to severe acne starts with a dose of 500 to 1000 milligrams a day, which is decreased as improvement occurs. Long-term, low-dose tetracycline therapy may be continued for many months to suppress acne. Higher doses may be prescribed for very severe acne.

Two synthetic derivatives of tetracycline used to treat acne are doxycycline and minocycline. Doxycycline proves especially effective in treating inflammatory acne. It can cause sun sensitivity in some patients. Minocyline has a long history of use in treating acne. It is often effective in treating acne that has not responded to other oral antibiotics. Minocycline also seems to produce fewer incidents of antibiotic resistance.

In most cases, topical medications and oral antibiotics may be used together. Studies have found that using topical benzoyl peroxide along with oral antibiotics may reduce the risk of developing antibiotic resistance. Antibiotics may cause side effects, such as an upset stomach, dizziness or skin discoloration. These drugs also increase the skin's sun sensitivity and may reduce the effectiveness of oral contraceptives.

### Topical Antimicrobials

Topical antimicrobials work to inhibit the *P. acnes* populations and are used to treat patients with mild to moderately severe inflammatory acne. They may be used alone or combined with a medication that works on another factor that leads to acne aside from *P. acnes.* A dermatologist can determine whether a topical antimicrobial is appropriate for a patient and if so which topical antimicrobial should be prescribed. Prescription topical antimicrobials used to treat acne vulgaris in the United States include:

Azelaic acid. Naturally occurring in the skin, azelaic acid is used to treat mild to moderate inflammatory and non-inflammatory acne. It is believed that azelaic acid clears acne by reducing the populations of *P. acnes,* decreasing the abnormal shedding of skin cells and reducing inflammation. This medication has also proven effective in treating the dark spots that develop in some acne patients with skin of color. Azaleic acid is well tolerated by most people and can be safely used for years. Side effects may include skin dryness and lightening of the skin where applied.

Benzoyl peroxide. Benzoyl peroxide works by killing *P. acnes.* However, it does not have anti-inflammatory abilities. It is available in a wide range of strengths and can be found as a gel, lotion, cleanser, cream and wash. Many acne preparations include benzoyl peroxide because research shows that benzoyl peroxide increases the effectiveness of some medicines, such as erythromycin and clindamycin. When used in combination with antibiotics, benzoyl peroxide also reduces the likelihood of a patient developing resistance to the antibiotic. The most common side effects are skin irritation, the potential to bleach hair and fabrics as well as possible allergic reaction.

Clindamycin. A semi-synthetic antibiotic, topical clindamycin has a long history of successfully treating acne. It works by reducing *P. acnes* and decreasing inflammation. In topical form, clindamycin has proven safe and is well tolerated. Skin dryness and irritation are possible side effects. It is important to use as directed to decrease bacterial resistance that can occur with antibiotic use.

Erythromycin. This topical antibiotic is active against a broad spectrum of bacteria, including *P. acnes.* Topical erythromycin, which is an antimicrobial and anti-inflammatory, is used primarily to treat acne. When topical erthyromycin is combined with benzoyl peroxide, the combination proves to be quite effective as the patient gets the effects of two antimicrobial agents. Like topical clindamycin, erythromycin may cause skin dryness and possible irritation. It is important to use as directed to decrease bacterial resistance that can occur with antibiotic use.

Sodium sulfacetamide. A topical antibiotic that inhibits *P. acnes* and opens clogged pores, sodium sulfacetamide is effective in treating inflammatory acne. Many products containing sodium sulfacetamide include sulfur. Some patients do not like the smell of the sulfur or its grittiness. Usually, the newer products that contain sulfur do not have these problems.

### Topical Retinoids

Prescribed to treat acne ranging from mild to moderately severe, topical retinoids are a derivative of vitamin A and considered a cornerstone in acne treatment. Retinoids work to unclog pores and prevent whiteheads and blackheads from forming. Topical retinoids can irritate the skin and increase sun sensitivity so it is important to use sun protection and follow the dermatologist's directions to maximize effectiveness. An added benefit in using topical retinoids is that they may help diminish the signs of aging, such as fine lines and wrinkles. Topical retinoids currently prescribed for acne treatment in the United States include:

Adapalene. A synthetic retinoid applied as a gel or cream, adapalene unclogs pores and possesses moderate to potent anti-inflammatory abilities. Improvement is usually seen in 8 to 12 weeks. Side effects include minor skin irritation and dryness.

Tazarotene. A synthetic retinoid available as a gel or cream, it works to keep the skin's pores clear and has proven effective in treating acne. This medication should not be used by women who are pregnant, and effective contraception is needed while taking tazarotene because the medication has produced birth defects in animals. Skin irritation is a possible side effect.

Tretinoin. The first retinoid developed for topical use, tretinoin is a natural retinoid. It works to gradually unclog pores and keep them unplugged. In the past, many patients found tretinoin too harsh for their skin; however, the newer forms are proving less irritating. Side effects include redness, scaling, dryness, itching and burning. If these occur, talk with the dermatologist who prescribed tretinoin as these side effects can be managed by adjusting the amount applied and when it is applied.

### Isotretinoin

For deep cysts, antibiotics may not be enough. Isotretinoin (Amnesteem, Claravis, Sotret) is a powerful medication available for scarring cystic acne or acne that does not respond to other treatments. This medicine is reserved for the most severe forms of acne. It is effective, but people who take it need close monitoring by a dermatologist because of the possibility of severe side effects. Isotretinoin is associated with severe birth defects, so it cannot be safely taken by pregnant women or women who may become pregnant during the course of treatment or within several weeks of concluding treatment. In fact, the drug carries such serious potential side effects that women of reproductive age must participate in a Food and Drug Administration-approved monitoring program to receive a prescription for the drug.

Isotretinoin commonly causes side effects - such as dry eyes, mouth, lips, nose and skin, as well as itching, nosebleeds, muscle aches, sun sensitivity and poor night vision. The drug may also increase the levels of triglycerides and cholesterol in the blood and may increase liver enzyme levels. In addition, isotretinoin may be associated with an increased risk of depression and suicide. Although this causal relationship has not been proved, doctors remain on alert for these signs in people who are taking isotretinoin. If you feel unusually sad or unable to cope while taking this drug, tell your doctor immediately.

### Oral contraceptives

Oral contraceptives, including a combination of norgestimate and ethinyl estradiol (Ortho Tri-Cyclen, Previfem, others), can improve acne in women by suppressing the overactive sebaceous glands and can be used as long-term acne therapy. However, oral contraceptives may cause other side effects - such as headaches, breast tenderness, nausea and depression. The most serious potential complication is a slightly increased risk of heart disease, high blood pressure and blood clots. Thus, oral contraceptives should not be prescribed to women who smoke, have a blood-clotting disorder, are older than 35 or have a history of migraine headaches-without the advice of a gynecologist.

### Laser and light-based therapies

Laser- and light-based therapies reach the deeper layers of skin without harming the skin's surface. Laser treatment is thought to damage the oil (sebaceous) glands, causing them to produce less oil. Light therapy targets the bacteria that cause acne inflammation. These therapies can also improve skin texture and lessen the appearance of scars. More research is needed to understand the most effective use of light and laser therapies in acne treatment, and experts currently recommend these approaches as stand-alone therapy only in people who cannot tolerate approved acne medications. These therapies may be uncomfortable and may cause temporary skin problems that mimic severe sunburn.

### Cosmetic procedures

Cosmetic procedures such as chemical peels and microdermabrasion may be helpful in controlling acne. These cosmetic procedures - which have traditionally been used to lessen the appearance of fine lines, sun damage and minor facial scars - are most effective when used in combination with other acne treatments. They may cause temporary, severe redness, scaling and blistering, and long-term discoloration of the skin.

### Interlesional corticosteroid injection

Interlesional corticosteroid injection is also available. When an acne cyst becomes severely inflamed, there is a good chance that it will rupture and scarring may result. To treat these severely inflamed cysts and prevent scarring, dermatologists may inject such cysts with a much-diluted corticosteroid. This lessens the inflammation and promotes healing. An interlesional corticosteroid injection works by "melting" the cyst over a period of 3 to 5 days.

### FORMS

The compositions presently disclosed may be administered, e.g., in capsule, tablet, powder, sachet, or any other form suitable for drug delivery. The dosage form can be of any shape, suitable for oral administration, such as spherical, oval, ellipsoidal, cube-shaped, regular, and/or irregular shaped. The dosage forms can be prepared according to processes known in the art and can include one or more additional pharmaceutically-acceptable excipients.

### Gelatin Capsule or Tablet

In some embodiments of the present disclosure, the compositions are in a capsule or a tablet form. The capsule wall-forming material may comprise, for example, gelatin or polysaccharides other than alginate. In at least one embodiment, the capsule is a gelatin or fish gelatin capsule. The capsules may be hard capsules or soft capsules. The capsule may be a cap-in-cap capsule.

An example of a "cap-in-cap" capsule is DuoCap^{™}, the only patented dual capsule system available to the pharmaceutical and nutraceutical industry. DuoCap involves specialist liquid-filling techniques using custom-designed filling equipment that allows the insertion of a pre-filled, smaller capsule into a larger, liquid-filled capsule. The smaller, inner capsule may contain either a liquid or semi-solid formulation and, according to the formulation or product requirements, either or both capsules may be of gelatin or HPMC composition and can be coated, if necessary. DuoCap^{™} has been successfully commercialised by Encap and is suited for both pharmaceutical and nutraceutical use.

When the dosage form is in the form of tablets, the tablets may be, for example, disintegrating tablets, fast dissolving tablets, effervescent tablets, fast melt tablets, and/or mini-tablets. Tablet formulations are described, for example, in patent publication WO 2006/000229. In some embodiments of the present disclosure, the tablets comprise Neusilin (e.g., magnesium aluminometasilicate).

When the capsule is a gelatin capsule, the gelatin may be chosen from type A gelatin, type B gelatin, or mixtures thereof. Gelatin is produced by destruction of secondary and, to a certain extent, higher structures in collagen (Babel, 1996). Gelatins may be of type A or B, depending on the kind of pre-treatment to which the collagenous tissue has been subjected. There are several processes by which collagen is processed to gelatin, but the two most common, as noted above, are the acidic and alkaline pre-treatments followed by extractions. Type A gelatin is obtained from animal skin, usually porcine skin, or hide, or from bovine, pork, and other animals, pre-treated with acid. The raw material is left in an acid soak for 10-30 hours depending on the nature of the collagenous stock. Type B gelatin is derived from collagenous raw materials subjected to alkaline pre-treatment. The alkali process is mainly used on bovine hide and bone collagfen sources where the animal is relatively old at slaughter (FRANCIS, F. J. 1999. Gelatin. Wiley Encyclopedia of Food Science and Technology. 2nd ed.: John Wiley & Sons). The collagenous stock is left in liming pits for periods of 3-10 weeks depending on the nature of the stock and the ambient temperature.

The capsules and/or tablets of the present disclosure may comprise at least one coating. Such coatings can delay the release of the capsule or tablet (e.g., release of the at least one fatty acid chosen from EPA and DHA) for a predetermined period. For example, the at least one coating may allow the dosage form to pass through the stomach without being subjected to stomach acid or digestive juices to provide for delayed release of the at least one fatty acid chosen from EPA and DHA outside of the stomach. In some embodiments, the capsules and/or tablets release less than 30% of the totalat least one fatty acid chosen from EPA and DHA in the stomach, such as less than 25%, less than 20%, less than 15%, or less than 10%.

In some embodiments, the at least one coating is chosen from enteric coatings, sub-layers, top-layers, and combinations thereof. The term "sub-layer" as used herein means a coating layer located between the capsule wall material (e.g., gelatin wall) or the tablet surface and an enteric coating. The term "top-layer" as used herein means a coating layer over an enteric coating covering the capsule wall material or the tablet surface. The chemical composition of sub-layers and top-layers may vary depending upon the overall composition of the capsule or tablet. Typical materials for the sub-layers and top-layers presently disclosed include film-forming agents such as polysaccharides, for example hydroxypropyl methyl cellulose.

In embodiments of the present disclosure, the capsules and/or tablets comprise at least one enteric coating. In some embodiments, the capsules and/or tablets comprise at least one enteric coating and at least one top-layer over the at least one enteric coating. In other embodiments, the capsules and/or tablets comprise at least one enteric coating and at least one sub-layer between the capsule wall or the tablet surface and the at least one enteric coating. In still other embodiments, the capsules and/or tablets comprise at least one enteric coating, at least one sub-layer between the capsule wall or the tablet surface, and at least one top-layer over the at least one enteric coating. In some embodiments, at least one of the sub-layer(s) and/or top-layer(s) comprises hydroxypropyl methyl cellulose.

In some embodiments, the at least one sub-layer comprises a sealant. Suitable sealants may comprise, for example, permeable or soluble agents such as hydroxypropyl methyl cellulose, hydroxypropyl cellulose, hydroxypropyl ethylcellulose, and xanthan gum. Other agents can be added to improve the processability of the sealant or barrier layer. Such agents include talc, colloidal silica, polyvinyl alcohol, titanium dioxide, micronized silica, fumed silica, glycerol monostearate, magnesium trisilicate and magnesium stearate, or a mixture thereof. The sealant or barrier layer can be applied from solution (e.g., aqueous) or suspension using any known means, such as a fluidized bed coater (e.g., Wurster coating) or pan coating system. Suitable sealants or barriers include, for example, Opadry® products such as Opadry® II available from Colorcon.

In some embodiments, the at least one coating is pH-independent. Coatings with pH-independent profiles generally erode or dissolve away after a predetermined period, and the period is generally directly proportional to the thickness of the coating. In other embodiments, the at least one coating is pH-dependent. Coatings with pH-dependent profiles can generally maintain their integrity while in the acid pH of the stomach, but erode or dissolve upon entering the more basic upper intestine. In some embodiments, the at least one coating is insoluble at a pH below about 5 and soluble at a pH above about 6.

Examples of coating materials suitable for the present disclosure include, but are not limited to, gelatin, film-forming agents, polymers, and copolymers. Examples of polymers and copolymers include, but are not limited to, acrylate-based polymers and copolymers (e.g., methacrylic acid, copolymers between methacrylic acid and methyl methacrylate, copolymers between methacrylic acid and methyl acrylate, copolymers between methacrylic acid and ethyl methacrylate, and copolymers between methacrylic acid and ethyl acrylate) and polysaccharide and/or cellulose-based polymers and copolymers (e.g., cellulose acetate phthalate, hydroxypropyl methyl cellulose phthalate, and hydroxypropyl methyl cellulose acetate succinate). Others polymers include, for example, polyvinyl acetate phthalate. Additional materials suitable for the at least one coating include pharmaceutically acceptable acidic compounds that may not dissolve at the low pH in the stomach, but may dissolve at higher pH in the lower part of the gastrointestinal system.

Commercially-available examples of polymers suitable for the present disclosure include EUDRAGIT® products from Evonik. EUDRAGIT® polymers are polymeric lacquer substances based on acrylates and/or methacrylates, and may be pH-independent or pH-dependent.

For example, EUDRAGIT® RL and EUDRAGIT® RS are acrylic resins comprising copolymers of acrylic and methacrylic acid esters with a low content of quaternary ammonium groups. The ammonium groups are present as salts and give rise to permeability of the lacquer films. EUDRAGIT® RL and EUDRAGIT® RS are freely permeable (RL) and slightly permeable (RS), respectively, independent of pH. The polymers swell in water and digestive juices, in a pH-independent manner. In the swollen state, they are permeable to water and to dissolved active compounds. Specific examples include EUDRAGIT® RL 30D, EUDRAGIT® RL PO, EUDRAGIT® RL 100, EUDRAGIT® RL 12,5, EUDRAGIT® RS 30D, EUDRAGIT® RS PO, EUDRAGIT® RS 100, and EUDRAGIT® RS 12,5. Additional examples of pH-independent polymers include EUDRAGIT® E 100, EUDRAGIT® E 12,5, and EUDRAGIT® E PO. In at least one embodiment of the present disclosure, the at least one coating comprises EUDRAGIT® RS 30D.

Further, for example, EUDRAGIT® L and EUDRAGIT® S are anionic polymers synthesized from methacrylic acid and methacrylic acid methyl ester. They are insoluble in acids and pure water, and become soluble in neutral to weakly alkaline conditions. The permeability of EUDRAGIT® L and EUDRAGIT S is pH dependent. Above pH 5.0, the polymers become increasingly permeable. Specific examples include EUDRAGIT® L100-55, EUDRAGIT® L30D-55, EUDRAGIT® L100, EUDRAGIT® L100 12,5, EUDRAGIT® S100, EUDRAGIT® S12,5, and EUDRAGIT® FS 30D. Additional examples of pH-dependent polymers include EUDRAGIT® E100, EUDRAGIT® E 12,5, and EUDRAGTT® PO. In at least one embodiment of the present disclosure, the at least one coating comprises EUDRAGIT® L100-55.

The at least one coating may comprise at least one plasticizer. Plasticizers may, for example to improve the mechanical properties of pH-sensitive materials of the at least one coating. Suitable plasticizers include, but are not limited to, triethyl citrate, triacetin, polyethylene glycols, propylene glycol, phthalates, sorbitol and glycerin. The amount of plasticizer may vary depending upon the chemical composition of the at least one coating and the chemical composition and size of the capsule or tablet. In some embodiments, for example, the amount of plasticizer ranges from about 10% to about 60% by weight of the at least one coating.

The amount of coating material or thickness of the at least one coating may vary depending upon the chemical compositions and number of different coating layers, and chemical composition, size, and shape of the capsule or the tablet. Generally speaking, the coating should be sufficient thick to prevent substantial release of the at least one fatty acid chosen from EPA and DHA in the stomach, but also not contribute significantly to the capsule or tablet size. In some embodiments of the present disclosure, the thickness of the at least one coating ranges from about 10 microns to about 2 mm, such as from about 20 microns to about 1 mm. In some embodiments, the at least one coating comprises from about 1% to about 50% of the dry capsule wall-forming material (e.g., gelatin).

The capsules according to the present disclosure may be manufactured in low oxygen conditions to inhibit oxidation during the manufacturing process. The capsules may be prepared, for example, by direct encapsulation using standard methods known in the art. Examples of such methods include, but are not limited to, simple coacervation methods (see, e.g., ES 2009346, EP 0052510, and EP 0346879), complex coacervation methods (see, e.g., GB 1393805), double emulsion methods (see, e.g., U.S. 4,652,441), simple emulsion methods (see, e.g., U.S. 5,445,832), and solvent evaporation methods (see, e.g., GB 2209937). Those methods may, for example, provide for continuous processing and flexibility of batch size. The present disclosure further provides for coating pre-prepared capsules (e.g., gelatin capsules comprising a fatty acid oil mixture). The coating of pre-prepared capsules may be performed, for example, by spraying such as using spray drying techniques or spraying into a coating pan comprising preformed capsules, or by dipping capsules into coating solutions.

In some embodiments of the present disclosure, the capsule fill content ranges from about 0.4 g to about 1.6 g. For example, in some embodiments, the capsule fill content ranges from about 0.4 g to about 1.3 g, or from about 0.6 g to about 1.2 g.

### Alginate Capsule

In at least one embodiment, the composition disclosed herein is in the form of a seamless capsule comprising a polysaccharide gel membrane outer surface shell comprising at least one alginate. In at least one embodiment, the polysaccharide gel membrane outer surface shell comprising at least one alginate encapsulates at least one emulsion comprises at least one oily phase, the at least one oily phase comprises the fatty acid oil mixture and at least one surfactant, the fatty acid oil mixture comprises at least 50% by weight of the emulsion, and the emulsion does not comprise marmelo mucilage.

As used herein, "alginate" includes alginic acid and/or pharmaceutically acceptable salts thereof, and refers generally to a copolymer comprising (1-4)-linked β-D-mannuronate (M) and its C-5 epimer α-L-guluronate (G) residues. Non-limiting examples of alginate salts suitable for the disclosure herein include alginate salts of calcium, strontium, barium, or aluminum. In one embodiment, alginate comprises all or in part M-alginate. In another embodiment, alginate comprises all or in part G-alginate. In another embodiment, alginate comprises a combination of M-alginate and G-alginate. In at least one embodiment, the alginate has a G content of at least 30% by weight. In other embodiments, the alginate has a content ranging from about 40% to about 80% by weight.

In at least one embodiment, the alginate shell achieves a time-release delivery of the at least one fatty acid chosen from EPA and DHA upon administration to a subject.

In some embodiments of the present disclosure, the alginate shell further comprises coloring agents, stabilizers, sweetening agents, plasticizers, and/or hardeners.

Other polymers contemplated as comprising the capsule shell include polyesters, polyacrylates, polycyanoacrylates, polysaccharides, polyethylene glycol, and mixtures thereof. Other polymers may include, for example, gelatin, carboxymethylcellulose alginates, carrageenans, pectins, ethyl cellulose hydroxypropyl methylcellulose, cellulose acetophthalate, hydroxypropyl methylcellulose phthalate, methylacrylicacid copolymers (Eudragit® Land S), dimethylaminoethylmethacrylate copolymers (Eudragit E), trimethylammoniumethylmethacrylate copolymers (e.g., Eudragit® RL and RS), polymers and copolymers of lactic and glycolic acids, and mixtures thereof. In one embodiment, the polymer comprises a plasticizer additive, such as, for example, but not limited to, triethyl citrate, butyl phthalate, and mixtures thereof. Other additives may optionally be incorporated to improve and/or facilitate the encapsulation process, such as, for example, fluidizing agents, such as talc.

The seamless capsules of the present disclosure may comprise at least one excipient. Thus, the at least one excipient may be chosen from, for example, colloidal silicon dioxide, crospovidone, lactose monohydrate, lecithin, microcrystalline cellulose, polyvinyl alcohol, povidone, sodium lauryl sulfate, sodium stearyl fumarate, talc, titanium dioxide, and xanthum gum.

Surfactants may be chosen from, for example, glycerol acetates and acetylated glycerol fatty acid esters, such as acetin, diacetin, triacetin, and/or mixtures thereof. Suitable acetylated glycerol fatty acid esters include, but are not limited to, acetylated monoglycerides, acetylated diglycerides, and/or mixtures thereof.

In addition, the surfactant may be chosen from glycerol fatty acid esters, such as, for example, those comprising a fatty acid component of about 6-22 carbon atoms. Glycerol fatty acid esters can be chosen from monoglycerides, diglycerides, triglycerides, and/or mixtures thereof. Suitable glycerol fatty acid esters include monoglycerides, diglycerides, medium chain triglycerides with fatty acids having about 6-12 carbons, and/or mixtures thereof. Capmul® MCM (medium chain mono- and di-glycerides) is an example.

The at least one surfactant may be chosen from propylene glycol esters. For example, propylene glycol esters include, but are not limited to, propylene carbonate, propylene glycol monoacetate, propylene glycol diacetate, propylene glycol fatty acid esters, acetylated propylene glycol fatty acid esters, propylene glycol fatty acid monoesters, propylene glycol fatty acid diesters, and mixtures thereof. Fatty acids may comprise, for example, about 6-22 carbon atoms. Examples of propylene glycol esters include, but are not limited to, propylene glycol monocaprylate (Capryol®), propylene glycol dicaprylate, propylene glycol dicaprate, propylene glycol dicaprylate/dicaprate, and mixtures thereof.

The at least one surfactant may be chosen from ethylene glycol esters, such as, for example, monoethylene glycol monoacetates, diethylene glycol esters, polyethylene glycol esters, and mixtures thereof. Additional examples include ethylene glycol monoacetates, ethylene glycol diacetates, ethylene glycol fatty acid monoesters, ethylene glycol fatty acid diesters, and mixtures thereof. In addition, the ethylene glycol esters may be chosen from polyethylene glycol fatty acid monoesters, polyethylene glycol fatty acid diesters, and mixtures thereof. Ethylene glycol esters may be obtained from the transesterification of polyethylene glycol and a triglyceride, a vegetable oil, and/or mixture thereof, and include, for example, those marketed as Labrafil® and Labrasol®: Polyoxyethylene-sorbitan-fatty acid esters (also called polysorbates), e.g., of from 4 to 25 alkylene moieties, for example monolauryl, trilauryl , palmityl, stearyl, and oleyl esters, including, for example, Tween®.

A group of suitable surfactants includes propylene glycol monocaprylate, mixtures of glycerol and polyethylene glycol esters of long fatty acids, polyethoxylated castor oils, nonylphenol ethoxylates (Tergitol®), glycerol esters, oleoyl macrogol glycerides, propylene glycol monolaurate, propylene glycol dicaprylate/dicaprate, polyethylene-polypropylene glycol copolymer, and polyoxyethylene sorbitan monooleate.

Hydrophilic solvents which may be used include, but are not limited to, alcohols, e.g., a water miscible alcohol such as absolute ethanol, and/or glycerol. Other alcohols include glycols, e.g., any glycol obtainable from an oxide such as ethylene oxide, e.g., 1,2-propylene glycol. Other non-limiting examples include polyols, e.g., a polyalkylene glycol; e.g., poly(C₂₋₃)alkylene glycol. One non-limiting example is a polyethylene glycol. The hydrophilic component may comprise an N-alkylpyrollidone, such as, but not ;limited to, N-(C₁₋₁₄alkyl)pyrollidone, e.g., N-methylpyrollidone, tri(C₁₋₄alkyl)citrate, e.g., triethylcitrate, dimethylisosorbide, (Csc13) alkanoic acid, e.g., caprylic acid and/or propylene carbonate. The hydrophilic solvent may comprise a main or sole component, e.g., an alcohol, e.g., C1-4-alcohol, e.g., ethanol, or alternatively a cocomponent, e.g., which may be chosen from partial lower ethers or lower alkanols. Suitable partial ethers include, for example, Transcutol® (which has the formula C2Hs-[O-(CH2)2]2-0H), Glycofurol® (also known as tetrahydrofurfuryl alcohol polyethylene glycol ether), or lower alkanols such as ethanol, such as, for example, glycerol acetates and acetylated glycerol fatty acid esters.

In at least one embodiment of the present disclosure, the capsules are seamless and comprise a polysaccharide gel membrane outer surface shell, and optionally a coating on said gel membrane. The polysaccharide gel membrane may be ionic. In some embodiments, the seamless capsules encapsulate an oily phase comprising the fatty acid oil mixture, water, and at least one surfactant. In some embodiments, the oily phase is an emulsion, such as an oil-in-water emulsion, a water-in-oil emulsion, or a water-in-oil-in-water emulsion. According to some embodiments of the present disclosure, the fatty acid oil mixture is present in an amount of at least 50% by weight of the emulsion, such as at least 55%, at least 60%, at least 65%, at least 70%, at least 75%, at least 80%, at least 85%, or even at least 90% by weight of said emulsion. In at least some embodiments, the seamless capsules do not comprise marmelo mucilage.

In some embodiments, the polysaccharide gel membrane further comprises at least one secondary film former chosen from cellulose acetate phthalate, cellulose acetate succinate, methyl cellulose phthalate, ethylhydroxycellulose phthalate, polyvinylacetatephtalate, polyvinylbutyrate acetate, vinyl acetate-maleic anhydride copolymer, styrene-maleic mono-ester copolymer, methyl acrylate-methacrylic acid copolymer, methacrylate-methacrylic acid-octyl acrylate copolymer, propylene glycol alginate, polyvinyl alcohol, carrageenans, pectins, chitosans, guar gum, gum acacia, sodium carboxymethylcellulose, hydroxypropylmethyl cellulose, hydroxypropylcellulose, methylcellulose, starches, and maltodextrins.

In some embodiments of the present disclosure, the polysaccharide gel membrane comprising the seamless capsules is an ionic gel membrane comprising at least one of alginate, propylene glycol alginate, and pectin. Said at least one of alginate, propylene glycol alginate, and pectin may be present in the form of a pharmaceutically acceptable salt, non-limiting examples of which include salts of calcium, strontium, barium, or aluminum. The ionic polysaccharide of the seamless capsules presently disclosed may comprise an alginate having a weight-average molecular weight ranging from about 20,000 Daltons to about 500,000 Daltons, such as from about 50,000 Daltons to about 500,000 Daltons, or about 100,000 Daltons to about 500,000 Daltons, or about 150,000 Daltons to about 500,000 Daltons, or about 150,000 Daltons to about 300,000 Daltons, or about 20,000 Daltons to about 200,000 Daltons, or from about 20,000 Daltons to about 100,000 Daltons, or from about 30,000 Daltons to about 80,000 Daltons, or from about 30,000 Daltons to about 60,000 Daltons, or even ranging from about 30,000 Daltons to about 40,000 Daltons. In some embodiments of the present disclosure, the ionic polysaccharide comprises a mixture of two alginate components, such as a mixture of (i) an alginate having a weight-average molecular weight ranging from about 30,000 Daltons to about 40,000 Daltons; and (ii) an alginate having a weight-average molecular weight ranging from about 150,000 Daltons to about 500,000 Daltons. In such embodiments, the ratio of (i) to (ii), (i):(ii), may range from about 0.1 to about 20, or about 1 to about 16.

The seamless capsules presently disclosed may be in a shape other than spherical. For example, in some embodiments of the present disclosure, the seamless capsules are oblong, oval, or cylindrical. The seamless capsules may be wet or dry.

The thickness of the polysaccharide gel film comprising the alginate shell of the seamless capsules presently disclosed may range from about 0.01 millimeter to about 50 millimeters. The polysaccharide gel film may be wet or dry. In some embodiments, the thickness of the polysaccharide gel film ranges from about 0.3 millimeters to about 4 millimeters. In some embodiments, the thickness of the polysaccharide gel film ranges from about 0.04 millimeters to about 0.5 millimeters.

The seamless capsules according to the present disclosure may have a wet capsule diameter ranging from about 0.5 millimeters to about 50 millimeters, such as about 1 millimeter to about 40 millimeters, wherein the gel membrane has a thickness ranging from about 0.1 millimeter to about 5 millimeters, such as about 0.3 millimeters to about 4 millimeters.

In some embodiments, the seamless capsule is dried, and the gel membrane is a dry polysaccharide gel film on the outer surface which constitutes up to 10% by weight of the dried seamless capsule. In some embodiments, the dry capsule has a diameter ranging from about 0.5 millimeters to about 35 millimeters, wherein the dry polysaccharide gel film has a thickness ranging from about 0.01 millimeters to about 5 millimeters. In some embodiments, the thickness of the dry polysaccharide gel film ranges from about 0.04 millimeters to about 0.5 millimeters.

According to at least one embodiment, an oil-in-water emulsion is encapsulated in seamless capsules for oral administration. The seamless capsules may also be known generally as softgels.

Seamless capsules of the present disclosure may be prepared, for example, by a method disclosed in WO 2003/084516, comprising: (a) preparing an emulsion comprising oil, water, an emulsifier, and at least one of a water-soluble monovalent metal salt, polyvalent metal salt, and an acid, wherein the oil is present in an amount of at least 50% by weight of the emulsion; and (b) adding at least one portion of the emulsion to an aqueous gelling bath comprised of at least one ionic polysaccharide, thereby encapsulating the at least one portion of the emulsion in a polysaccharide gel membrane, and optionally (c) drying the resulting capsules.

In one embodiment of the present disclosure, the at least one polyvalent metal salt is calcium chloride (CaCI₂) and the at least one ionic polysaccharide is alginate. In one embodiment, the alginate is all or in part M-alginate. In one embodiment, the alginate is all or in part G-alginate. In one embodiment, the alginate is a mixture of M-alginate and G-alginate.

### Methods or Uses

The present disclosure further encompasses methods of improving at least one parameter associated with acne vulgaris and/or eczema in a subject in need thereof. The food supplement, dietary supplement, nutritional supplement, OTC supplement, medical food, or pharmaceutical grade supplement compositions presently disclosed may be administered, e.g., in capsule, tablet or any other suitable form for drug delivery, to a subject for at least one of:
- affecting inflammatory lesions on the face and/or trunk in relation to acne;
- affecting non-inflammatory lesions on the face and/or trunk in relation to acne;
- affecting nodules and/or cysts on the face and/or trunk in relation to acne;
- affecting anti-inflammatory response in relation to acne in adolescents;
- affecting erythema in relation to eczema;
- affecting scaling in relation to eczema; and
- affecting pruritus in relation to eczema.

In addition, the composition disclosed herein may be administered to a subject with acne who are or are not symptom-free to reduce a dosage of usual (common) treatment of acne.

The present disclosure also encompasses the use of a composition chosen from a food supplement, dietary supplement, nutritional supplement, over-the-counter (OTC) supplement, medical food, pharmaceutical grade supplement for at least one aspect of skin health chosen from: improving skin health in subjects with acne, maintaining skin health in subjects with acne who are symptom-free, improving skin health in subjects with eczema, and maintaining skin health in subjects with eczema who are symptom-free comprising: a fatty acid oil mixture comprising from about 25% to about 100% of at least one fatty acid chosen from eicosapentaenoic acid (EPA) and docosahexaenoic acid (DHA), by weight of the fatty acid oil mixture, wherein the EPA and DHA are in a form chosen from ethyl ester, triglyceride, and free fatty acid; and vitamin D.

In at least one embodiment, affecting skin health is chosen from improving skin health, helping preserve normal skin health, improving facial skin health, and helping preserve normal facial skin health.

In at least one embodiment, affecting anti-inflammatory response in relation to acne is chosen from helping preserve normal inflammatory response in relation to sebum production and stimulating normal resolution of skin inflammation in relation to sebaceous gland production.

The total daily dosage of the fatty acid oil mixture may range in amount from about 1 g to about 6 g, such as from about 1 g to about 3 g or from about 1.5 g to about 3 g. The total daily dosage of the vitamin D may range in amount from about 400 to about 4000 International Units (IU), from about 1000 to about 4000 International Units (IU), or from about 2000 to about 4000 International Units (IU).

The administration may be oral or any other form of administration that provides a dosage of fatty acids, e.g., omega-3 fatty acids, and vitamin D to a subject, such as a human. For example, the compositions presently disclosed may be administered as capsules and/or tablets in from 1 to 10 dosages, such as from 1 to 4 times a day, such as once, twice, three times, or four times per day, and further for example, once, twice or three times per day.

In at least one embodiment, the food supplement, dietary supplement, nutritional supplement, OTC supplement, medical food, or pharmaceutical grade supplement composition is administered to the subject in need thereof daily for a period of at least 3 months.

In at least one embodiment, the food supplement, dietary supplement, nutritional supplement, OTC supplement, medical food, or pharmaceutical grade supplement composition comprises a first composition comprising the fatty acid oil mixture and a second composition comprising the vitamin D, wherein the two compositions are administered to the subject in need thereof in any order, and wherein the first composition and the second composition can each be in the form of any of a gelatin capsule or tablet, a sachet, or a seamless capsule comprising a polysaccharide gel membrane outer surface shell comprising at least one alginate.

In another embodiment, the food supplement, dietary supplement, nutritional supplement, OTC supplement, medical food, or pharmaceutical grade supplement composition comprises a single composition comprising the fatty acid oil mixture and the vitamin D.

In some embodiments, the food supplement, dietary supplement, nutritional supplement, OTC supplement, medical food, or pharmaceutical grade supplement composition is in a gelatin capsule or a tablet form comprising a fatty acid oil mixture comprising from about 75% to about 90% of at least one fatty acid chosen from eicosapentaenoic acid (EPA) and docosahexaenoic acid (DHA), by weight of the fatty acid oil mixture, wherein the EPA and DHA are in a form chosen from ethyl ester and triglyceride and are present at a EPA:DHA weight ratio ranging from about 1:8 to about 1:6, and vitamin D. Moreover, in at least one such embodiment, the daily dosage ranges from about 1.500 to about 3.000 mg/g fatty acid oil mixture and from about 1000 to about 4000 IU Vitamin D.

### Formulations

In some embodiments, the composition in a gelatin capsule or a tablet form is a food supplement, dietary supplement, nutritional supplement, OTC supplement, medical food, or pharmaceutical grade supplement composition comprising a fatty acid oil mixture comprising from about 25% to about 100% of at least one fatty acid chosen from eicosapentaenoic acid (EPA) and docosahexaenoic acid (DHA), by weight of the fatty acid oil mixture, wherein the EPA and DHA are in a form chosen from ethyl ester and triglyceride; and vitamin D, wherein the gelatin capsule or the tablet comprises at least one coating.

In other embodiments, the composition in a seamless capsule comprising a polysaccharide gel membrane outer surface shell comprising at least one alginate form is a food supplement, dietary supplement, nutritional supplement, OTC supplement, medical food, or pharmaceutical grade supplement composition comprising a fatty acid oil mixture comprising from about 50% to about 85% of at least one fatty acid chosen from eicosapentaenoic acid (EPA) and docosahexaenoic acid (DHA), by weight of the fatty acid oil mixture, wherein the EPA and DHA are in a form chosen from ethyl ester and triglyceride; and vitamin D, wherein the gelatin capsule or the tablet comprises at least one coating.

In still other embodiments, the composition in a sachet form is a food supplement, dietary supplement, nutritional supplement, OTC supplement, medical food, or pharmaceutical grade supplement composition comprising a fatty acid oil mixture comprising from about 45% to about 85% of at least one fatty acid chosen from eicosapentaenoic acid (EPA) and docosahexaenoic acid (DHA), by weight of the fatty acid oil mixture, wherein the EPA and DHA are in a form chosen from ethyl ester and triglyceride; and vitamin D.

In other embodiments, the composition in a seamless capsule comprising a polysaccharide gel membrane outer surface shell comprising at least one alginate form is a food supplement, dietary supplement, nutritional supplement, OTC supplement, medical food, or pharmaceutical grade supplement composition comprising a fatty acid oil mixture comprising from about 70% to about 90% of at least one fatty acid chosen from eicosapentaenoic acid (EPA) and docosahexaenoic acid (DHA), by weight of the fatty acid oil mixture, wherein the EPA and DHA are in a form chosen from ethyl ester and triglyceride and are present at a EPA: DHA weight ratio ranging from about 1:8 to about 1:6, and vitamin D, wherein the gelatin capsule or the tablet comprises at least one coating. Moreover, in at least one such embodiment, the daily dosage ranges from about 1.5 to about 3 mg/g fatty acid oil mixture and from about 1000 to about 4000 IU Vitamin D in the application acne or eczema.

The following examples are intended to illustrate the present disclosure without, however, being limiting in nature. It is understood that the skilled artisan will envision additional embodiments consistent with the disclosure provided herein.

### EXAMPLE 1

Examples of fatty acid oil mixtures which may be used in the compositions, methods, and uses disclosed herein include those listed in the following table:

| **Fatty acid oil mixture** | **EPA EE or TG content (mg/g)** | **DHA EE or TG content (mg/g)** | **Total EPA and DHA (mg/g)** | **Vitamin D content (International Units (IU) 1.25(OH)₂D₃)** |
|---|---|---|---|---|
| PRONOVAPURE 150:600 | 125-175mg/g | 575-625 mg/g | 700-800 mg/g | 2000-4000 |
| PRONOVAPURE 200:500 | Min 200 mg/g EPA-EE or EPA-TG | Minimum 500 mg/g EPA-EE or EPA-TG | Minimum 700 mg/g | 2000-4000 |
| PRONOVA PURE 360:240 | Minimum 360 mg/g | Minimum 240 mg/g | Minimum 700 mg/g | 2000-4000 |
| MAXOMEGA 600:300 | Minimum 600 mg/g | Minimum 300 mg/g | Minimum 900 mg/g | 2000-4000 |
| MAXOMEGA high DHA | | Minimum 750 mg/g | | 2000-4000 |

### EXAMPLE 2

In separate (per clinical diagnosis) double-blind, prospective clinical trials, patients with clinically-defined acne or eczema may be randomly allocated to consume either the following mixed fatty acid oil compositions together with 2000 or 4000 (IU) Vitamin D, or placebo control capsules. The capsules administered may be further enteric coated.

| **Fatty acid oil mixture (w%)** | **EPA EE or TG content (w%)** | **DHA EE/TG content (w%)** | **Omega-3 (w%)** | **Vitamin D content (International Units (IU) 1,25(OH)₂D₃)** |
|---|---|---|---|---|
| Pronova DHA 1070EE | 8-13 % | 65-75 % | 75-90% | 2000-4000 |
| Pure DHA (>95% DHA-EE) | less than 5 % | >95 % DHA-EE | at least 95 % | 2000-4000 |
| Ultra Pure DHA (>97% DHA-EE and no EPA) | no EPA | >97 % DHA-EE | at least 97 % | 2000-4000 |
| Pronova 70:10 | 65-75 w% | 8-13 w% | 75-90 % | 2000-4000 |

| | | | | |
|---|---|---|---|---|
| EE = ethyl ester; TG = triglyceride | | | | |

As another example, the fatty acid oil mixture can be Pronova PURE 200:500 EE or TG and a high DHA-EE or TG having at least 900 mg/g DHA-EE or TG.

### EXAMPLE 3

In an *in vitro* assay, synergistic effects of various combinations of eicosapentaenoic acid (EPA), docosahexaenoic acid (DHA), and 1.25(OH)₂D₃, upon keratinocyte activation via measuring levels of selected cytokines and growth factors may be studied.

A dose response curve of the effects of titrated concentrations of 1.25(OH)₂D₃ (ranging from 10⁻¹² to 10⁻⁶ M) may be established and subsequently tested in combination with either EPA or DHA, each present at 2, 5, 10, 15, 30 and 50 uM.

## Claims

1. A food supplement, dietary supplement, nutritional supplement, over-the-counter (OTC) supplement, medical food, or pharmaceutical grade supplement composition for acne vulgaris and/or eczema comprising: a fatty acid oil mixture comprising from about 25 percent to about 100 percent of at least one fatty acid chosen from eicosapentaenoic acid (EPA) and docosahexaenoic acid (DHA), by weight of the fatty acid oil mixture, wherein the EPA and DHA are in a form chosen from ethyl ester, triglyceride, and free fatty acid; and vitamin D.

2. The composition according to claim 1, wherein the composition comprises a first composition comprising the fatty acid oil mixture and a second composition comprising the vitamin D.

3. The composition according to claim 1, wherein the composition comprises a single composition comprising the fatty acid oil mixture and the vitamin D.

4. The composition according to claim 1, wherein the fatty acid oil mixture comprises a higher concentration by weight of DHA than EPA.

5. The composition according to claim 1, wherein the EPA and DHA are in ethyl ester or triglyceride form.

6. The composition according to claim 1, wherein the fatty acid oil mixture further comprises at least one other fatty acid other than EPA and DHA in a form chosen from ethyl ester and triglyceride.

7. The composition according to claim 6, wherein the at least one other fatty acid is chosen from a-linolenic acid (ALA), heneicosapentaenoic acid (HPA), docosapentaenoic acid (DPA), eicosatetraenoic acid (ETA), octadecatetraenoic acid, and combinations thereof.

8. The composition according to claim 1, wherein the fatty acid oil mixture is derived from at least one oil chosen from marine oil, algae oil, plant-based oil, and microbial oil.

9. The composition according to claim 1, wherein the at least one fatty acid comprises EPA and DHA present at a EPA:DHA weight ratio ranging from about 1:10 to about 10:1, from about 1:8 to about 8:1, from about 1:7 to about 7:1, from about 1:6 to about 6:1, from about 1:5 to about 5:1, from about 1:4 to about 4:1, from about 1:3 to about 3.1, from about 1:2 to about 2:1, or from about 1:1 to about 2:1.

10. The composition according to claim 1, wherein the fatty acid oil mixture comprises at least about 200 mg/g EPA chosen from ethyl ester and triglyceride form, at least about 500 mg/g DHA chosen from ethyl ester and triglyceride form, wherein the fatty acid oil mixture comprises at least 700 mg/g omega-3 fatty acids and 2000-4000 IU vitamin D.

11. The composition according to claim 1, further comprising at least one antioxidant.

12. The composition according to claim 11, wherein the at least one antioxidant is chosen from a-tocopherol (vitamin E), calcium disodium EDTA, alpha tocoferyl acetates, butylhydroxytoluenes (BHT), butylhydroxyanisoles (BHA), ascorbic acid and pharmaceutically acceptable salts and esters thereof, propyl gallate, citric acid and pharmaceutically acceptable salts thereof, malic acid and pharmaceutically acceptable salts thereof, and sulfite salts and mixtures thereof.

13. The composition according to claim 1, wherein the composition further comprises at least one vitamin other than vitamin D.

14. The composition according to claim 1, wherein the composition is in the form of a gelatin capsule, a cap-in-cap capsule, a tablet, a powder, a sachet, or a seamless capsule comprising a polysaccharide gel membrane outer surface shell comprising at least one alginate.

15. The composition according to claim 14, wherein the gelatin capsule or tablet releases less than 30 percent of the total at least one fatty acid chosen from EPA and DHA in the stomach.

16. The composition according to claim 14, wherein the polysaccharide gel membrane outer surface shell comprising at least one alginate encapsulates at least one emulsion comprising at least one oily phase, the at least one oily phase comprises the fatty acid oil mixture and at least one surfactant, the fatty acid oil mixture comprises at least 50 percent by weight of the emulsion, and the emulsion does not comprise marmelo mucilage.

17. The composition according to claim 15, wherein the fatty acid oil mixture comprises at least 80 percent by weight of the emulsion.

18. The composition according to claim 15, wherein the at least one emulsion further comprises from about 0.1 to about 3 percent surfactant by weight and from about 0.1 to about 6 percent gelling salt by weight, each with respect to the total weight of the at least one emulsion.

19. The composition according to claim 1, wherein the fatty acid oil mixture comprises from about 80 mg/g to about 130 mg/g EPA ethyl ester or triglyceride and from about 650 mg/g to about 750 mg/g DHA ethyl ester or triglyceride.

20. The composition according to claim 1, wherein vitamin D is chosen from vitamin D2 (ergocalciferol) and vitamin D₃ (cholecalciferol).

21. The composition according to claim20, wherein the vitamin D is suspended or dissolved in the fatty acid oil mixture, encapsulated or micro-encapsulated in the fatty acid oil mixture, sprayed on a capsule encapsulating the fatty acid oil mixture, or a cap-in-cap capsule.

22. The composition according to claim 1, wherein the composition is administered to a subject in need thereof in a total daily dosage of the fatty acid oil mixture ranging in an amount from about 1 g about 6 g.

23. The composition according to claim 1, wherein the composition is administered to a subject in need thereof in a total daily dosage of the vitamin D ranging in an amount from about 400 to about 6000 International Units (IU), from about 1000 to about 4000 IU, or from about 2000 to about 4000 IU.

24. The composition according to claim 1, wherein the fatty acid oil mixture comprises at least one omega-3 fatty acid chosen from those defined in the European Pharmacopoeia Omega-3 Triglycerides and the European Pharmacopoeia Omega-3 acid Ethyl Esters 60.

25. The composition according to claim 1, wherein the composition is administered to reduce a dosage of usual (common) treatment of acne.

26. The composition according to claim 1, wherein the composition improves skin health in subjects with acne and/or maintains skin health in subjects with acne who are symptom-free, and/or improves skin health in subjects with eczema, and/or maintains skin health in subjects with eczema who are symptom-free.

27. Use of a composition chosen from a food supplement, dietary supplement, nutritional supplement, over-the-counter (OTC) supplement, medical food, and pharmaceutical grade supplement for at least one of improving skin health in subjects with acne, maintaining skin health in subjects with acne who are symptom-free, improving skin health in subjects with eczema, and/or maintaining skin health in subjects with eczema who are symptom-free, the composition comprising: a fatty acid oil mixture comprising from about 25 percent to about 100 percent of at least one fatty acid chosen from eicosapentaenoic acid (EPA) and docosahexaenoic acid (DHA), by weight of the fatty acid oil mixture, wherein the EPA and DHA are in a form chosen from ethyl ester, triglyceride, and free fatty acid; and vitamin D.

28. The use according to claim 27, wherein the improvement associated with acne vulgaris and/or eczema is chosen from: • affecting inflammatory lesions on the face and/or trunk in relation to acne; • affecting non-inflammatory lesions on the face and/or trunk in relation to acne; • affecting nodules and/or cysts on the face and/or trunk in relation to acne; • affecting anti-inflammatory response in relation to acne in adolescents; • affecting erythema in relation to eczema; • affecting scaling in relation to eczema; and • affecting pruritus in relation to eczema.

29. The use according to claim 27 or 28, wherein the effect on skin health is chosen from improving skin health, helping preserve normal skin health, improving facial skin health, and helping preserve normal facial skin health.

30. The use according to any of the claims 27 to 29, wherein the effect on anti-inflammatory response in relation to acne is chosen from helping preserve normal inflammatory response in relation to sebum production and stimulating normal resolution of skin inflammation in relation to sebaceous gland production.

31. The use according to any of the claims 27 to 30, wherein the food supplement, dietary supplement, nutritional supplement, OTC supplement, medical food, or pharmaceutical grade supplement composition is administered to the subject daily for a period of at least 3 months.

32. The use according to claim 27, wherein the fatty acid oil mixture comprises at least about 200 mg/g EPA chosen from ethyl ester and triglyceride form, at least about 500 mg/g DHA chosen from ethyl ester and triglyceride form, wherein the fatty acid oil mixture comprises at least 700 mg/g omega-3 fatty acids and 2000-4000 IU vitamin D.

33. The use according to claim 27, wherein the composition is in the form of a gelatin capsule, a cap-in-cap capsule, a tablet, a powder, a sachet, or a seamless capsule comprising a polysaccharide gel membrane outer surface shell comprising at least one alginate.

34. The use according to claim 27, wherein the at least one fatty acid and the vitamin D in combination achieve a synergistic effect.

## Patentansprüche

1. Nahrungsergänzungsmittel, Nahrungszusatz, Nährstoffanreicherung, rezeptfreies (over the counter, OTC) Ergänzungsmittel, medizinisches Nahrungsmittel oder Ergänzungsmittelzusammensetzung pharmazeutischer Qualität für Akne vulgaris und/oder Ekzeme, umfassend: eine Fettsäure-Öl-Mischung, umfassend von etwa 25 Prozent bis etwa 100 Prozent des Gewichts der Fettsäure-Öl-Mischung von mindestens einer Fettsäure ausgewählt aus Eicosapentaensäure (EPA) und Docosahexaensäure (DHA), wobei die EPA und DHA in einer Form, ausgewählt aus Ethylester, Triglycerid und freier Fettsäure, vorliegen; und Vitamin D.

2. Zusammensetzung nach Anspruch 1, wobei die Zusammensetzung eine erste Zusammensetzung umfasst, umfassend die Fettsäure-Öl-Mischung und eine zweite Zusammensetzung umfassend das Vitamin D.

3. Zusammensetzung nach Anspruch 1, wobei die Zusammensetzung einer einzige Zusammensetzung umfasst, umfassend die Fettsäure-Öl-Mischung und das Vitamin D.

4. Zusammensetzung nach Anspruch 1, wobei die Fettsäure-Öl-Mischung auf das Gewicht bezogen eine höhere Konzentration von DHA als von EPA umfasst.

5. Zusammensetzung nach Anspruch 1, wobei die EPA und DHA in der Form von Ethylester oder Triglycerid vorliegen.

6. Zusammensetzung nach Anspruch 1, wobei die Fettsäure-Öl-Mischung mindestens eine weitere, von EPA und DHA verschiedene, Fettsäure in einer Form ausgewählt aus Ethylester und Triglycerid umfasst.

7. Zusammensetzung nach Anspruch 6, wobei die mindestens einer weitere Fettsäure ausgewählt ist aus a-Linolensäure (ALA), Heneicosapentaensäure (HPA), Docosapentaensäure (DPA), Eicosatetraensäure (ETA), Octadecatetraensäure und Kombinationen davon.

8. Zusammensetzung nach Anspruch 1, wobei die Fettsäure-Öl-Mischung von mindestens einem Öl, ausgewählt aus Meeresöl, Algenöl, Öl auf Pflanzenbasis und mikrobiellem Öl, abgeleitet ist.

9. Zusammensetzung nach Anspruch 1, wobei mindestens eine Fettsäure EPA und DHA, vorliegend bei einem EPA:DHA-Gewichtsverhältnis im Bereich von etwa 1:10 bis etwa 10:1, von etwa 1:8 bis etwa 8:1, von etwa 1:7 bis etwa 7:1, von etwa 1:6 bis etwa 6:1, von etwa 1:5 bis etwa 5:1, von etwa 1:4 bis etwa 4:1, von etwa 1:3 bis etwa 3.1, von etwa 1:2 bis etwa 2:1 oder von etwa 1:1 bis etwa 2:1 umfasst.

10. Zusammensetzung nach Anspruch 1, wobei die Fettsäure-Öl-Mischung mindestens etwa 200 mg/g EPA, ausgewählt aus der Ethylester- und Triglyceridform, mindestens etwa 500 mg/g DHA, ausgewählt aus der Ethylester- und Triglyceridform umfasst, wobei die Fettsäure-Öl-Mischung mindestens 700 mg/g Omega-3-Fettsäuren und 2000-4000 IU Vitamin D umfasst.

11. Zusammensetzung nach Anspruch 1, ferner umfassend mindestens ein Antioxidationsmittel.

12. Zusammensetzung nach Anspruch 11, wobei das mindestens eine Antioxidationsmittel ausgewählt ist aus a-Tocopherol (Vitamin E), Calciumdinatrium-EDTA, alpha-Tocopherylacetaten, Butylhydroxytoluolen (BHT), Butylhydroxyanisolen (BHA), Ascorbinsäure und pharmazeutisch verträglichen Salzen und Estern davon, Propylgallat, Zitronensäure und pharmazeutisch verträglichen Salzen davon, Äpfelsäure und pharmazeutisch verträglichen Salzen davon, und Sulfitsalzen und Mischungen davon.

13. Zusammensetzung nach Anspruch 1, wobei die Zusammensetzung ferner mindestens ein, von Vitamin D verschiedenes, Vitamin umfasst.

14. Zusammensetzung nach Anspruch 1, wobei die Zusammensetzung in der Form einer Gelatinekapsel, einer "Kapsel-in-Kapsel"-Kapsel, einer Tablette, einem Pulver, einem Beutel oder einer nahtlosen Kapsel umfassend eine Polysaccharidgelmembran-Außenoberflächenschale, umfassend mindestens ein Alginat, vorliegt.

15. Zusammensetzung nach Anspruch 14, wobei die Gelatinekapsel oder Tablette weniger als 30 Prozent der gesamten mindestens einen Fettsäure, ausgewählt aus EPA und DHA, im Magen freisetzt.

16. Zusammensetzung nach Anspruch 14, wobei die mindestens ein Alginat umfassende Polysaccharidgelmembran-Außenoberflächenschale mindestens eine Emulsion, umfassend mindestens eine ölige Phase, einkapselt, wobei die mindestens eine ölige Phase die Fettsäure-Öl-Mischung und mindestens ein Tensid umfasst, wobei die Fettsäure-Öl-Mischung mindestens 50 Gewichtsprozent der Emulsion umfasst und die Emulsion keinen Quittenschleimstoff umfasst.

17. Zusammensetzung nach Anspruch 15, wobei die Fettsäure-Öl-Mischung auf das Gewicht bezogen mindestens 80 Prozent der Emulsion umfasst.

18. Zusammensetzung nach Anspruch 15, wobei die mindestens eine Emulsion ferner von etwa 0,1 bis etwa 3 Gewichtsprozent Tensid und von etwa 0,1 bis etwa 6 Gewichtsprozent Geliersalz umfasst, jeweils bezogen auf das Gesamtgewicht der mindestens einen Emulsion.

19. Zusammensetzung nach Anspruch 1, wobei die Fettsäure-Öl-Mischung von etwa 80 mg/g bis etwa 130 mg/g EPA-Ethylester oder -Triglycerid und von etwa 650 mg/g bis etwa 750 mg/g DHA-Ethylester oder -Triglycerid umfasst.

20. Zusammensetzung nach Anspruch 1, wobei Vitamin D ausgewählt ist aus Vitamin D2 (Ergocalciferol) und Vitamin D₃ (Cholecalciferol).

21. Zusammensetzung nach Anspruch 20, wobei das Vitamin D in der Fettsäure-Öl-Mischung suspendiert oder gelöst, in der Fettsäure-Öl-Mischung eingekapselt oder mikro-eingekapselt, auf eine die Fettsäure-Öl-Mischung einkapselnde Kapsel gesprüht oder eine "Kapsel-in-Kapsel"-Kapsel ist.

22. Zusammensetzung nach Anspruch 1, wobei die Zusammensetzung einem Patienten, der dieser Bedarf, in einer täglichen Gesamtdosis der Fettsäure-Öl-Mischung im Bereich einer Menge von etwa 1 g bis etwa 6 g verabreicht wird.

23. Zusammensetzung nach Anspruch 1, wobei die Zusammensetzung einem Patienten, der dieser Bedarf, in einer täglichen Gesamtdosis des Vitamin D in einer Menge von etwa 400 bis etwa 6000 Internationalen Einheiten (IU), von etwa 1000 bis etwa 4000 IU oder von etwa 2000 bis etwa 4000 IU verabreicht wird.

24. Zusammensetzung nach Anspruch 1, wobei die Fettsäure-Öl-Mischung mindestens eine Omega-3-Fettsäure, ausgewählt aus denjenigen definiert im Europäischen Arzneibuch Omega-3-Triglyceride und dem Europäischen Arzneibuch Omega-3-Ethylester 60, umfasst.

25. Zusammensetzung nach Anspruch 1, wobei die Zusammensetzung verabreicht wird, um eine Dosis üblicher (gewöhnlicher) Aknebehandlung zu verringern.

26. Zusammensetzung nach Anspruch 1, wobei die Zusammensetzung die Gesundheit der Haut bei Patienten mit Akne und/oder Gesundheit der Haut bei Patienten mit Akne, die symptomfrei sind, verbessert, und/oder Gesundheit der Haut bei Patienten mit Ekzemen verbessert, und/oder Gesundheit der Haut bei Patienten mit Ekzemen, die symptomfrei sind, aufrecht erhält.

27. Verwendung einer Zusammensetzung ausgewählt aus einem Nahrungsergänzungsmittel, einem Nahrungszusatz, einer Nährstoffanreicherung, einem rezeptfreien (OTC) Ergänzungsmittel, einem medizinischen Nahrungsmittel und Ergänzungsmittel pharmazeutischer Qualität für mindestens eines von Verbessern der Gesundheit der Haut bei Patienten mit Akne, aufrecht erhalten der Gesundheit der Haut bei Patienten mit Akne, die symptomfrei sind, Verbessern der Gesundheit der Haut bei Patienten mit Ekzemen und/ oder aufrecht erhalten von der Gesundheit der Haut bei Patienten mit Ekzemen, die symptomfrei sind, wobei die Zusammensetzung umfasst: eine Fettsäure-Öl-Mischung, umfassend von etwa 25 Prozent bis etwa 100 Prozent des Gewichts der Fettsäure-Öl-Mischung von mindestens einer Fettsäure, ausgewählt aus Eicosapentaensäure (EPA) und Docosahexaensäure (DHA), wobei die EPA und DHA in einer Form, ausgewählt aus Ethylester, Triglycerid und freier Fettsäure, vorliegen; und Vitamin D.

28. Verwendung nach Anspruch 27, wobei die mit Akne vulgaris und/oder Ekzemen assoziierte Verbesserung ausgewählt ist aus: • Beeinflussen entzündlicher Läsionen im Gesicht und/oder auf dem Rumpf in Bezug auf Akne; • Beeinflussen nicht-entzündlicher Läsionen im Gesicht und/oder auf dem Rumpf in Bezug auf Akne; • Beeinflussen von Knötchen und/oder Zysten im Gesicht und/oder auf dem Rumpf in Bezug auf Akne; • Beeinflussen entzündungshemmender Reaktion in Bezug auf Akne bei Heranwachsenden; • Beeinflussen von Erythemas in Bezug auf Ekzeme; • Beeinflussen von Abschuppen in Bezug auf Ekzeme; und • Beeinflussen von Pruritus in Bezug auf Ekzeme.

29. Verwendung nach Anspruch 27 oder 28, wobei die Wirkung auf die Gesundheit der Haut ausgewählt ist aus Verbessern der Gesundheit der Haut, Helfen, normale Gesundheit der Haut zu bewahren, Verbessern der Gesundheit der Haut des Gesichts, und Helfen, normale Gesundheit der Haut des Gesicht zu bewahren.

30. Verwendung nach einem der Ansprüche 27 bis 29, wobei die Wirkung der entzündungshemmenden Reaktion in Bezug auf Akne ausgewählt ist aus Helfen, normale entzündliche Reaktion in Bezug auf Sebumproduktion zu bewahren und dem Stimulieren normaler Resolution von Hautentzündung in Bezug auf die Talgdrüsenproduktion.

31. Verwendung nach einem der Ansprüche 27 bis 30, wobei das Nahrungsergänzungsmittel, der Nahrungszusatz, die Nährstoffanreicherung, das OTC-Ergänzungsmittel, das medizinisches Nahrungsmittel oder die Ergänzungsmittelzusammensetzung pharmazeutischer Qualität dem Patienten täglich für einen Zeitraum von mindestens 3 Monaten verabreicht wird.

32. Verwendung nach Anspruch 27, wobei die Fettsäure-Öl-Mischung mindestens etwa 200 mg/g EPA, ausgewählt aus der Ethylester- und Triglyceridform, mindestens etwa 500 mg/g DHA, ausgewählt aus der Ethylester- und Triglyceridform umfasst, wobei die Fettsäure-Öl-Mischung mindestens 700 mg/g Omega-3-Fettsäuren und 2000-4000 IU Vitamin D umfasst.

33. Verwendung nach Anspruch 27, wobei die Zusammensetzung in der Form einer Gelatinekapsel, einer "Kapsel-in-Kapsel"-Kapsel, einer Tablette, einem Pulver, einem Beutel oder einer nahtlosen Kapsel, umfassend eine Polysaccharidgelmembran-Außenoberflächenschale, umfassend mindestens ein Alginat, vorliegt.

34. Verwendung nach Anspruch 27, wobei die mindestens eine Fettsäure und das Vitamin D in Kombination eine synergistische Wirkung erzielen.

## Revendications

1. Composition de complément alimentaire, de complément diététique, de complément nutritionnel, de complément en vente libre (OTC), d'aliment médical ou de supplément de qualité pharmaceutique pour l'acné vulgaire et/ou l'eczéma comprenant : un mélange d'huiles d'acides gras comprenant d'environ 25 pour cent à environ 100 pour cent d'au moins un acide gras choisi parmi l'acide eicosapentaénoïque (EPA) et l'acide doco-sahexaénoïque (DHA), en poids du mélange d'huiles d'acides gras, dans laquelle l'EPA et le DHA sont sous une forme choisie parmi ester éthylique, triglycéride et acide gras libre ; et de la vitamine D.

2. Composition selon la revendication 1, où la composition comprend une première composition comprenant le mélange d'huiles d'acides gras et une deuxième composition comprenant la vitamine D.

3. Composition selon la revendication 1, où la composition comprend une composition unique comprenant le mélange d'huiles d'acides gras et la vitamine D.

4. Composition selon la revendication 1, dans laquelle le mélange d'huiles d'acides gras comprend une concentration plus élevée en poids de DHA que d'EPA.

5. Composition selon la revendication 1, dans laquelle l'EPA et le DHA sont sous une forme d'ester éthylique ou de triglycéride.

6. Composition selon la revendication 1, dans laquelle le mélange d'huiles d'acides gras comprend en outre au moins un autre acide gras, autre que EPA et DHA sous une forme choisie parmi un ester éthylique et un triglycéride.

7. Composition selon la revendication 6, dans laquelle l'au moins un autre acide gras est choisi parmi l'acide α-linolénique (ALA), l'acide héneicosapentaénoïque (HPA), l'acide docosapentaénoïque (DPA), l'acide eicosatetraénoïque (ETA), l'acide octadécatétraénoïque et leurs combinaisons.

8. Composition selon la revendication 1, dans laquelle le mélange d'huiles d'acides gras est dérivé d'au moins une huile choisie parmi une huile marine, une huile d'algues, une huile à base de plante et une huile microbienne.

9. Composition selon la revendication 1, dans laquelle l'au moins un acide gras comprend de l'EPA et du DHA présents à un rapport pondéral EPA:DHA allant d'environ 1:10 à environ 10:1, d'environ 1:8 à environ 8:1, d'environ 1:7 à environ 7:1, d'environ 1:6 à environ 6:1, d'environ 1:5 à environ 5:1, d'environ 1:4 à environ 4:1, d'environ 1:3 à environ 3:1, d'environ 1:2 à environ 2:1 ou d'environ 1:1 à environ 2:1.

10. Composition selon la revendication 1, dans laquelle le mélange d'huiles d'acides gras comprend au moins environ 200 mg/g d'EPA choisi parmi une forme ester éthylique et triglycéride, au moins environ 500 mg/g de DHA choisi parmi une forme ester éthylique et triglycéride, dans laquelle le mélange d'huiles d'acides gras comprend au moins 700 mg/g d'acides gras oméga-3 et 2000 à 4000 UI de vitamine D.

11. Composition selon la revendication 1, comprenant en outre au moins un antioxydant.

12. Composition selon la revendication 11, dans laquelle l'au moins un antioxydant est choisi parmi l'a-tocophérol (vitamine E), l'EDTA disodique de calcium, des acétates d'alpha-tocoféryle, des butylhydroxytoluènes (BHT), des butylhydroxyanisoles (BHA), l'acide ascorbique et les sels et esters acceptables sur le plan pharmaceutique de celui-ci, le gallate de propyle, l'acide citrique et les sels acceptables sur le plan pharmaceutique de celui-ci, l'acide malique et les sels acceptables sur le plan pharmaceutique de celui-ci et des sels sulfite et leurs mélanges.

13. Composition selon la revendication 1, où la composition comprend en outre au moins une vitamine autre que la vitamine D.

14. Composition selon la revendication 1, où la composition se présente sous la forme d'une capsule en gélatine, une capsule « cap-in-cap », un comprimé, une poudre, un sachet ou une capsule sans joint comprenant une enveloppe de surface externe à membrane en gel de polysaccharide comprenant au moins un alginate.

15. Composition selon la revendication 14, dans laquelle la capsule de gélatine ou le comprimé libère moins de 30 pour cent du total de l'au moins un acide gras choisi parmi EPA et DHA dans l'estomac.

16. Composition selon la revendication 14, dans laquelle l'enveloppe de surface externe à membrane en gel de polysaccharide comprenant au moins un alginate encapsule au moins une émulsion comprenant au moins une phase huileuse, l'au moins une phase huileuse comprend le mélange d'huiles d'acides gras et au moins un agent tensioactif, le mélange d'huiles d'acides gras constitue au moins 50 pour cent en poids de l'émulsion et l'émulsion ne comprend pas de mucilage de marmelo.

17. Composition selon la revendication 15, dans laquelle le mélange d'huiles d'acides gras constitue au moins 80 pour cent en poids de l'émulsion.

18. Composition selon la revendication 15, dans laquelle l'au moins une émulsion comprend en outre d'environ 0,1 à environ 3 pour cent d'agent tensioactif en poids et d'environ 0,1 à environ 6 pour cent de sel gélifiant en poids, chacun par rapport au poids total de l'au moins une émulsion.

19. Composition selon la revendication 1, dans laquelle le mélange d'huiles d'acides gras comprend d'environ 80 mg/g à environ 130 mg/g d'ester éthylique ou triglycéride d'EPA et d'environ 650 mg/g à environ 750 mg/g d'ester éthylique ou triglycéride de DHA.

20. Composition selon la revendication 1, dans laquelle la vitamine D est choisie parmi la vitamine D2 (ergocalciférol) et la vitamine D₃ (cholécalciférol).

21. Composition selon la revendication 20, dans laquelle la vitamine D est mise en suspension ou dissoute dans le mélange d'huiles d'acides gras, en-capsulée ou micro-encapsulée dans le mélange d'huiles d'acides gras, pulvérisée sur une capsule encapsulant le mélange d'huiles d'acides gras ou est une capsule « cap-in-cap ».

22. Composition selon la revendication 1, où la composition est administrée à un sujet qui en a besoin en une posologie totale quotidienne du mélange d'huiles d'acides gras s'élevant à une quantité allant d'environ 1 g à environ 6 g.

23. Composition selon la revendication 1, où la composition est administrée à un sujet qui en a besoin en une posologie totale quotidienne de la vitamine D s'élevant à une quantité allant d'environ 400 à environ 6000 unités internationales (UI), d'environ 1000 à environ 4000 UI ou d'environ 2000 à environ 4000 UI.

24. Composition selon la revendication 1, dans laquelle le mélange d'huiles d'acides gras comprend au moins un acide gras oméga-3 choisi parmi ceux définis dans les triglycérides oméga-3 de la Pharmacopée européenne et les esters éthyliques 60 d'acide oméga-3 de la Pharmacopée européenne.

25. Composition selon la revendication 1, où la composition est administrée pour réduire une posologie d'un traitement habituel (courant) de l'acné.

26. Composition selon la revendication 1, où la composition améliore la santé de la peau chez des sujets présentant de l'acné et/ou maintient la santé de la peau chez des sujets présentant de l'acné qui sont dépourvus de symptôme et/ou améliore la santé de la peau chez des sujets présentant de l'eczéma et/ou maintient la santé de la peau chez des sujets présentant de l'eczéma qui sont dépourvus de symptôme.

27. Utilisation d'une composition choisie parmi un complément alimentaire, un complément diététique, un complément nutritionnel, un complément en vente libre (OTC), un aliment médical et un supplément de qualité pharmaceutique pour au moins un effet parmi l'amélioration de la santé de la peau chez des sujets présentant de l'acné, le maintien de la santé de la peau chez des sujets présentant de l'acné qui sont dépourvus de symptôme, l'amélioration de la santé de la peau chez des sujets présentant de l'eczéma et/ou le maintien de la santé de la peau chez des sujets présentant de l'eczéma qui sont dépourvus de symptôme, la composition comprenant : un mélange d'huiles d'acides gras comprenant d'environ 25 pour cent à environ 100 pour cent d'au moins un acide gras choisi parmi l'acide eicosapentaénoïque (EPA) et l'acide docosahexaénoïque (DHA), en poids du mélange d'huiles d'acides gras, dans laquelle l'EPA et le DHA sont sous une forme choisie parmi un ester éthylique, un triglycéride et un acide gras libre ; et de la vitamine D.

28. Utilisation selon la revendication 27, dans laquelle l'amélioration associée à l'acné vulgaire et/ou à l'eczéma est choisie parmi : • affecter des lésions inflammatoires sur le visage et/ou le torse en rapport avec l'acné ; • affecter des lésions non inflammatoires sur le visage et/ou le torse en rapport avec l'acné ; • affecter des nodules et/ou kystes sur le visage et/ou le torse en rapport avec l'acné ; • affecter une réponse anti-inflammatoire en rapport avec l'acné chez des adolescents ; • affecter un érythème en rapport avec l'eczéma ; • affecter une desquamation en rapport avec l'eczéma ; et • affecter un prurit en rapport avec l'eczéma.

29. Utilisation selon la revendication 27 ou 28, dans laquelle l'effet sur la santé de la peau est choisi parmi améliorer la santé de la peau, aider à préserver la santé normale de la peau, améliorer la santé de la peau du visage et aider à préserver la santé normale de la peau du visage.

30. Utilisation selon l'une quelconque des revendications 27 à 29, dans laquelle l'effet sur la réponse anti-inflammatoire en rapport avec l'acné est choisi parmi aider à préserver une réponse inflammatoire normale par rapport à la production de sébum et stimuler une résolution normale d'une inflammation de la peau par rapport à la production des glandes sébacées.

31. Utilisation selon l'une quelconque des revendications 27 à 30, dans laquelle la composition de complément alimentaire, de complément diététique, de complément nutritionnel, de complément en vente libre (OTC), d'aliment médical ou de complément de qualité pharmaceutique est administrée au sujet quotidiennement pendant une période d'au moins 3 mois.

32. Utilisation selon la revendication 27, dans laquelle le mélange d'huiles d'acides gras comprend au moins environ 200 mg/g d'EPA choisi parmi une forme ester éthylique et triglycéride, au moins environ 500 mg/g de DHA choisi parmi une forme ester éthylique et triglycéride, dans laquelle le mélange d'huiles d'acides gras comprend au moins 700 mg/g d'acides gras oméga-3 et 2000 à 4000 UI de vitamine D.

33. Utilisation selon la revendication 27, où la composition se présente sous la forme d'une capsule en gélatine, une capsule « cap-in-cap », un comprimé, une poudre, un sachet ou une capsule sans joint comprenant une enveloppe de surface externe à membrane en gel de polysaccharide comprenant au moins un alginate.

34. Utilisation selon la revendication 27, dans laquelle l'au moins un acide gras et la vitamine D réalisent en combinaison un effet synergique.
